(19) Europäisches Patentamt
European Patent Office
Office européen des brevets

(11) EP 4 015 502 A1

(12) EUROPEAN PATENT APPLICATION
published in accordance with Art. 153(4) EPC

(43) Date of publication:
**22.06.2022 Bulletin 2022/25**

(21) Application number: **20852540.2**

(22) Date of filing: **11.08.2020**

(51) International Patent Classification (IPC):
***C07C 271/10*** *(2006.01)* ***C07C 271/20*** *(2006.01)*
***C07C 271/24*** *(2006.01)* ***C08F 290/06*** *(2006.01)*
***C08G 18/67*** *(2006.01)* ***A61K 6/30*** *(2020.01)*
***A61K 6/882*** *(2020.01)* ***A61K 6/887*** *(2020.01)*
***A61K 6/889*** *(2020.01)* ***A61K 6/893*** *(2020.01)*
***A61K 6/90*** *(2020.01)*

(52) Cooperative Patent Classification (CPC):
**A61K 6/30; A61K 6/882; A61K 6/887; A61K 6/889; A61K 6/893; A61K 6/90; C07C 271/10; C07C 271/20; C07C 271/24; C08F 290/06; C08G 18/67**

(86) International application number:
**PCT/JP2020/030607**

(87) International publication number:
**WO 2021/029406 (18.02.2021 Gazette 2021/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.08.2019 JP 2019148911**
**31.10.2019 JP 2019199162**

(71) Applicant: **Mitsui Chemicals, Inc.**
**Tokyo 105-7122 (JP)**

(72) Inventor: **KAKINUMA, Naoyuki**
**Sodegaura-shi, Chiba 299-0265 (JP)**

(74) Representative: **Hoffmann Eitle**
**Patent- und Rechtsanwälte PartmbB**
**Arabellastraße 30**
**81925 München (DE)**

# (54) URETHANE ALLYL COMPOUND, MONOMER COMPOSITION, MOLDED ARTICLE, COMPOSITION FOR DENTAL MATERIAL, AND DENTAL MATERIAL

(57) A urethane allyl compound having a urethane bond and an allyloxy group.

EP 4 015 502 A1

FIG.1A

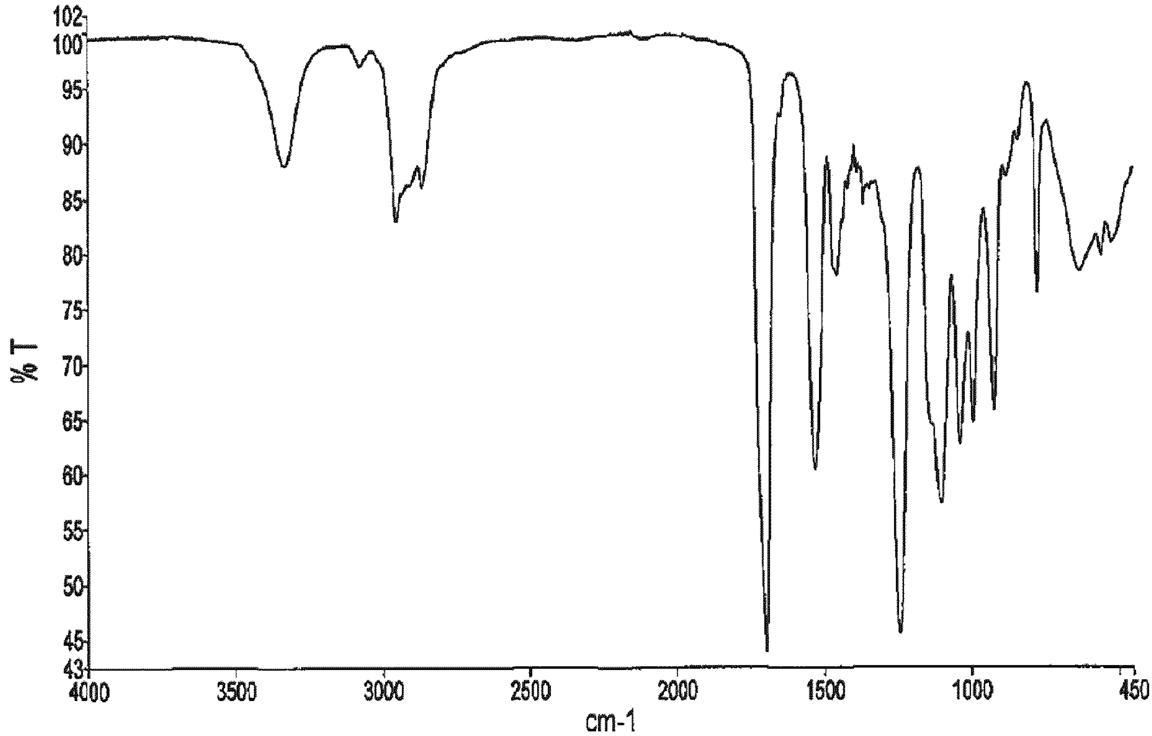
102
100
95
90
85
80
75
70
65
60
55
50
45
43
% T
4000
3500
3000
2500
2000
1500
1000
450
cm-1

FIG.1B

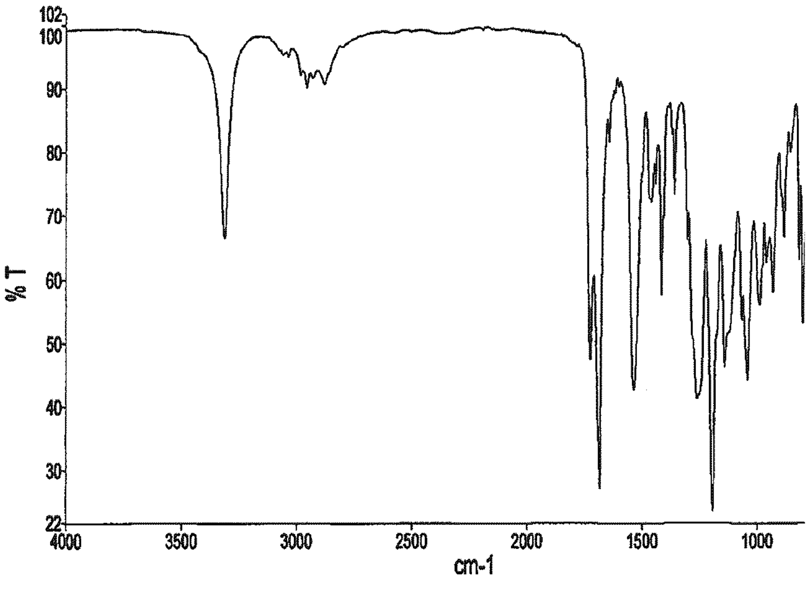
102
100
90
80
70
60
50
40
30
22
% T
4000
3500
3000
2500
2000
1500
1000
cm-1

## Description

### Technical Field

The present disclosure relates to a urethane allyl compound, a monomer composition, a molded body, a composition for a dental material, and a dental material.

### Background Art

Conventionally, polymerizable monomers represented by (meth)acrylate compounds and allyl compounds have been widely used in various fields such as paints, printing plate making, optical materials, and dental materials, by utilizing characteristics such as favorable curability and transparency.
Among them, in the field of dental materials, polymerizable monomers have been widely used for dental restorative materials such as dental composite resins used for restoration of caries, breakage, and the like of natural teeth, various dental adhesives used for bonding the dental composite resin and teeth, artificial teeth, denture base materials, and the like.
For example, Patent Document 1 discloses a crown and bridge composition containing a diallyl phthalate prepolymer, a diallyl phthalate monomer, and a polymerization initiator.
Patent Document 1: Japanese Patent Application Laid-Open (JP-A) No. S62-149608

## SUMMARY OF INVENTION

### Technical Problem

In a case in which a cured product is formed using a diallyl phthalate monomer as disclosed in Patent Document 1, there is room for improvement in mechanical strength such as breaking strength and breaking energy.
The disclosure has been made in view of the above problems, and provides a urethane allyl compound capable of forming a cured product excellent in breaking strength and breaking energy, a monomer composition containing the urethane allyl compound, a molded body which is a cured product of the monomer composition, a composition for a dental material containing the monomer composition, and a dental material which is a cured product of the composition for a dental material.

### Solution to Problem

Means for solving the above problems is as follows.

<1> A urethane allyl compound having a urethane bond and an allyloxy group.
<2> The urethane allyl compound according to <1>, which is a urethane allyl compound (X) containing no (meth)acryloyloxy group or a urethane allyl (meth)acrylate compound (Y) containing a (meth)acryloyl group.
<3> The urethane allyl compound according to <2>,
in which the urethane allyl compound (X) is a reaction product of an iso(thio)cyanate compound (A) having two or more iso(thio)cyanate groups and an alcohol compound (B) having an allyloxy group, and the urethane allyl (meth)acrylate compound (Y) is a reaction product of an iso(thio)cyanate compound (A) having two or more iso(thio)cyanate groups, an alcohol compound (B) having an allyloxy group, and an alcohol compound (C) having a (meth)acryloyloxy group.
<4> The urethane allyl compound according to <3>, in which the iso(thio)cyanate compound (A) having two or more iso(thio)cyanate groups contains at least one selected from the group consisting of m-xylylene diisocyanate, 1,3-tetramethylxylylene diisocyanate, a mixture of 2,2,4-trimethylhexamethylene diisocyanate and 2,4,4-trimethylhexamethylene diisocyanate, a mixture of 2,5-bis(isocyanatomethyl) bicyclo[2.2.1]heptane and 2,6-bis(isocyanatomethyl) bicyclo[2.2.1]heptane, and isophorone diisocyanate.
<5> The urethane allyl compound according to <3> or <4>, in which the alcohol compound (B) having an allyloxy group is at least one selected from the group consisting of the following compounds (B-1), (B-2), and (B-3):

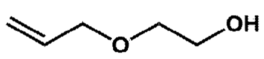

(B−1)

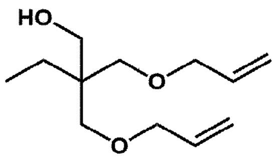

(B−2)

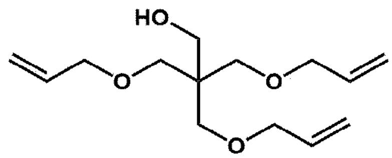

(B−3)

<6> The urethane allyl compound according to any one of <3> to <5>, in which the alcohol compound (C) having a (meth)acryloyloxy group contains at least one selected from the group consisting of 2-hydroxyethyl (meth)acrylate, 2-hydroxypropyl (meth)acrylate, 2-hydroxybutyl (meth)acrylate, 4-hydroxybutyl (meth)acrylate, 2-hydroxy-3-phenoxypropyl (meth)acrylate, and 1,4-cyclohexanedimethanol mono(meth)acrylate.

<7> The urethane allyl compound according to any one of <1> to <6>, which is a compound represented by the following general formula (XI) or the following general formula (Y1).

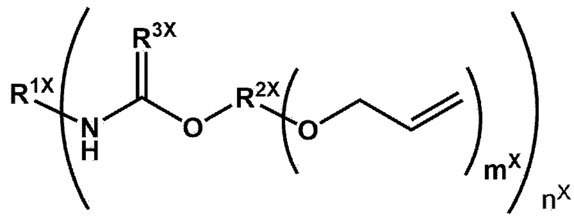

(X1)

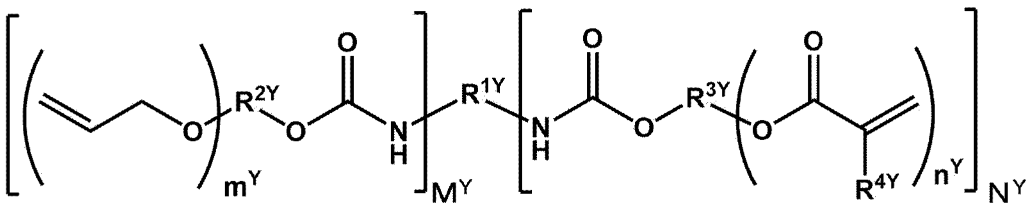

(Y1)

wherein, in formula (XI), $R^{1X}$ is a residue obtained by removing $n^X$ iso(thio)cyanate groups from the iso(thio)cyanate compound (A) having $n^X$ iso(thio)cyanate groups, $R^{2X}$ is a residue obtained by removing $m^X$ allyloxy groups and one hydroxy group from the alcohol compound (B) having $m^X$ allyloxy groups, and $R^{3X}$ is an oxygen atom or a sulfur atom. $n^X$ is an integer of 2 or more, and $m^X$ is an integer of 1 or more,

and in formula (Y1), $R^{1Y}$ is a residue obtained by removing $M^Y + N^Y$ iso(thio)nate groups from the iso(thio)cyanate compound (A) having two or more iso(thio)cyanate groups,

$R^{2Y}$ is a residue obtained by removing $m^Y$ allyloxy groups and one hydroxy group from the alcohol compound (B) having an allyloxy group,

$R^{3Y}$ is a residue obtained by removing $n^Y$ (meth)acryloyloxy groups and one hydroxy group from the alcohol compound (C) having a (meth)acryloyloxy group, and

$R^{4Y}$ is a hydrogen atom or a methyl group, $n^Y$ is an integer of 1 or more, $m^Y$ is an integer of 1 or more, $M^Y$ is an integer from 1 to 3, $N^Y$ is an integer from 1 to 3, and $M^Y + N^Y$ is an integer from 2 to 4.

<8> The urethane allyl compound according to any one of <1> to <7>, in which the urethane allyl compound has a molecular weight of from 200 to 1,500.

<9> A monomer composition containing the urethane allyl compound according to any one of claims <1> to <8> and a (meth)acrylate compound (D).

<10> A monomer composition containing the urethane allyl compound according to any one of <1> to <8>, which is for dental use.

<11> A molded body which is a cured product of the monomer composition according to <9> or <10>.

<12> A composition for a dental material, containing the monomer composition according to <9> or <10> and a

polymerization initiator.

<13> The composition for a dental material according to <12>, further containing a filler.

<14> A dental material which is a cured product of the composition for a dental material according to <12> or <13>.

Advantageous Effects of Invention

According to the disclosure, it is possible to provide a urethane allyl compound capable of forming a cured product excellent in breaking strength and breaking energy, a monomer composition containing the urethane allyl compound, a molded body which is a cured product of the monomer composition, a composition for a dental material containing the monomer composition, and a dental material which is a cured product of the composition for a dental material.

The urethane allyl compound may be a urethane allyl (meth)acrylate compound.

BRIEF DESCRIPTION OF DRAWINGS

Fig. 1A shows an IR spectrum of a urethane allyl compound (A-1) obtained in Example 1A.
Fig. 2A shows an IR spectrum of a urethane allyl compound (A-2) obtained in Example 2A.
Fig. 3 A shows an IR spectrum of a urethane allyl compound (A-3) obtained in Example 3A.
Fig. 4A shows an IR spectrum of a urethane allyl compound (A-4) obtained in Example 4A.
Fig. 5A shows an IR spectrum of a urethane allyl compound (A-5) obtained in Example 5A.
Fig. 6A shows an IR spectrum of a urethane allyl compound (A-6) obtained in Example 6A.
Fig. 7A shows an IR spectrum of a urethane allyl compound (A-7) obtained in Example 7A.
Fig. 8A shows an IR spectrum of a urethane allyl compound (A-8) obtained in Example 8A.
Fig. 9A shows an IR spectrum of a urethane allyl compound (A-9) obtained in Example 9A.
Fig. 10A shows an IR spectrum of a urethane allyl compound (A-10) obtained in Example 10A.
Fig. 11A shows an IR spectrum of a urethane allyl compound (A-11) obtained in Example 11A.
Fig. 1B shows an IR spectrum of a urethane allyl (meth)acrylate compound (B-1) obtained in Example 1B.
Fig. 2B shows an IR spectrum of a urethane allyl (meth)acrylate compound (B-2) obtained in Example 2B.
Fig. 3B shows an IR spectrum of a urethane allyl (meth)acrylate compound (B-3) obtained in Example 3B.
Fig. 4B shows an IR spectrum of a urethane allyl (meth)acrylate compound (B-4) obtained in Example 4B.
Fig. 5B shows an IR spectrum of a urethane allyl (meth)acrylate compound (B-5) obtained in Example 5B.
Fig. 6B shows an IR spectrum of a urethane allyl (meth)acrylate compound (B-6) obtained in Example 6B.
Fig. 7B shows an IR spectrum of a urethane allyl (meth)acrylate compound (B-7) obtained in Example 7B.
Fig. 8B shows an IR spectrum of a urethane allyl (meth)acrylate compound (B-8) obtained in Example 8B.
Fig. 9B shows an IR spectrum of a urethane allyl (meth)acrylate compound (B-9) obtained in Example 9B.
Fig. 10B shows an IR spectrum of a urethane allyl (meth)acrylate compound (B-10) obtained in Example 10B.
Fig. 11B shows an IR spectrum of a urethane allyl (meth)acrylate compound (B-11) obtained in Example 11B.
Fig. 12B shows an IR spectrum of a urethane allyl (meth)acrylate compound (B-12) obtained in Example 12B.
Fig. 13B shows an IR spectrum of a urethane allyl (meth)acrylate compound (B-13) obtained in Example 13B.

DESCRIPTION OF EMBODIMENTS

Hereinafter, modes for carrying out the disclosure will be described in detail. However, the disclosure is not limited to the following embodiments. In the following embodiments, the components (also including element steps and the like) are not essential unless otherwise specified. The same applies to numerical values and ranges thereof, and the disclosure is not limited thereto.

In the disclosure, a numerical range indicated using "to" includes numerical values described before and after "to" as a minimum value and a maximum value, respectively.

In the numerical ranges described in stages in the disclosure, the upper limit value or the lower limit value described in one numerical range may be replaced with the upper limit value or the lower limit value of the numerical range described in another stage. In addition, in the numerical range described in the disclosure, the upper limit value or the lower limit value of the numerical range may be replaced with a value shown in Examples.

In the disclosure, the "(meth)acryloyl" means acryloyl or methacryloyl, and the "(meth)acrylate" means acrylate or methacrylate.

In the disclosure, the "iso(thio)cyanate" means isocyanate or isothiocyanate.

In the disclosure, the "urethane bond" includes, for example, a bond formed by reaction between an isocyanate

group of the isocyanate compound (A) and a hydroxy group of the alcohol compound (B), and a bond formed by reaction between an isothiocyanate group of the isothiocyanate compound (A) and a hydroxy group of the alcohol compound (B).

In the disclosure, in the case of referring to the amount of each component in the composition, in a case where there are a plurality of substances corresponding to each component in the composition, it means the total amount of the plurality of substances present in the composition unless otherwise specified.

[Urethane Allyl Compound]

The urethane allyl compound of the disclosure has a urethane bond and an allyloxy group. The urethane allyl compound of the disclosure can form a cured product excellent in breaking strength and breaking energy. It is presumed that since the allyloxy group has higher polymerizability than the (meth)acryloyl group, the reaction rate in the cured product is high, the cured product has a strong structure, and the urethane allyl compound has a urethane bond, and therefore the breaking strength and breaking energy of the cured product are improved.

Furthermore, the urethane allyl compound of the disclosure can form a cured product having a low polymerization shrinkage rate.

In the urethane allyl compound of the disclosure, the number of urethane bonds and the number of allyloxy groups are not particularly limited. For example, the number of urethane bonds is preferably 2 or 3, and more preferably 2. The number of allyloxy groups is preferably from 2 to 9, and more preferably from 2 to 6.

The urethane allyl compound described above is preferably a reaction product of an iso(thio)cyanate compound (A) having two or more iso(thio)cyanate groups (hereinafter, also referred to as "iso(thio)cyanate compound (A)".) and an alcohol compound (B) having an allyloxy group (hereinafter, also referred to as an "alcohol compound (B)".). More specifically, the urethane allyl compound is more preferably a reaction product having a urethane bond obtained by reacting the iso(thio)cyanate group of the iso(thio)cyanate compound (A) with the hydroxy group of the alcohol compound (B). The urethane allyl compound is further preferably a reaction product having two or more urethane bonds obtained by reacting all the iso(thio)cyanate groups of the iso(thio)cyanate compound (A) with the hydroxy group of the alcohol compound (B).

The urethane allyl compound of the disclosure is preferably a compound represented by the following general formula (XI).

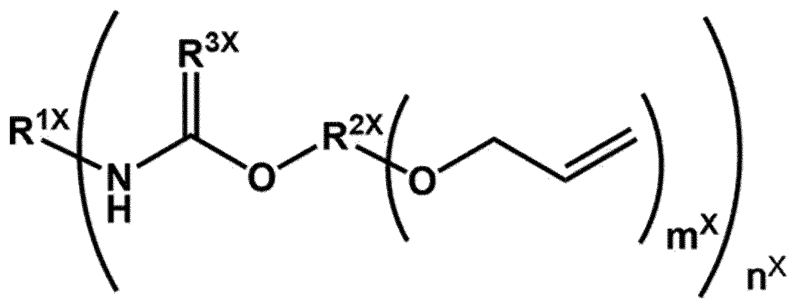

(X1)

wherein $R^{1X}$ is a residue obtained by removing $n^X$ iso(thio)cyanate groups from the iso(thio)cyanate compound (A) having $n^X$ iso(thio)cyanate groups, $R^{2X}$ is a residue obtained by removing $m^X$ allyloxy groups and one hydroxy group from the alcohol compound (B) having $m^X$ allyloxy groups, and $R^{3X}$ is an oxygen atom or a sulfur atom. $n^X$ is an integer of 2 or more, and $m^X$ is an integer of 1 or more.

A plurality of $R^{2X}$s and $R^{3X}$s may be the same or different.

In a case in which $R^{1X}$ is a residue obtained by removing $n^X$ isocyanate groups from the isocyanate compound (A) having $n^X$ isocyanate groups, $R^{3X}$ is an oxygen atom, and in a case in which $R^{1X}$ is a residue obtained by removing $n^X$ isothiocyanate groups from the isothiocyanate compound (A) having $n^X$ isothiocyanate groups, $R^{3X}$ is a sulfur atom.

$m^X$ is preferably from 1 to 3, and more preferably 2 or 3 from the viewpoint of more excellent breaking energy and a smaller polymerization shrinkage rate when a cured product is obtained using a urethane allyl compound.

$n^X$ is preferably 2 or 3, and more preferably 2.

Hereinafter, an iso(thio)cyanate compound (A) having two or more iso(thio)cyanate groups and an alcohol compound (B) having an allyloxy group, which can be used for producing the urethane allyl compound of the disclosure,

and reaction conditions thereof will be described.

(Iso(thio)cyanate Compound (A))

The iso(thio)cyanate compound (A) is a compound having two or more iso(thio)cyanate groups, and is preferably a compound in which A iso(thio)cyanate groups are bonded to an A-valent linking group. Here, A is an integer of 2 or more, preferably from 2 to 4, more preferably 2 or 3, and still more preferably 2.

In a case in which A is 2, the divalent linking group is not particularly limited, and examples thereof include alkylene groups, arylene groups, -C(=O)-, $-SO_2-$, -NR- (R represents a hydrogen atom or an alkyl group having from 1 to 10 carbon atoms, and a hydrogen atom is preferable), and groups including any combination thereof. Among them, an alkylene group, an arylene group, and any combination thereof are preferable as the divalent linking group.

The number of carbon atoms in the alkylene group is preferably from 1 to 20, more preferably from 1 to 10, and still more preferably from 1 to 5. The alkylene group may have a substituent or may be unsubstituted. The alkylene group may be any of linear, branched, cyclic, or any combination thereof. The cyclic alkylene group may be either monocyclic or polycyclic. In a case in which the alkylene group is cyclic, the number of carbon atoms in the alkylene group is preferably from 6 to 18, more preferably from 6 to 14, and still more preferably from 6 to 10. Examples of the linear or branched alkylene group include an ethylene group, an n-propylene group, an isopropylene group, an n-butylene group, an isobutylene group, a sec-butylene group, a tert-butylene group, an n-pentylene group, an isopentylene group, a neopentylene group, and a tert-pentylene group. Examples of the cyclic alkylene group include groups having a norbornane structure and groups having an isophorone structure.

The number of carbon atoms in the arylene group is preferably from 6 to 18, more preferably from 6 to 14, and still more preferably from 6 to 10. Specific examples of the arylene group include an o-phenylene group, an m-phenylene group, a p-phenylene group, and divalent condensed polycyclic aromatic ring groups in which two or more aromatic rings are condensed.

In Formula (1) described above, $R^1$ may be one substituent selected from residues obtained by removing all iso(thio)nate groups from the iso(thio)cyanate compound (A), and it is preferable that any of a plurality of $R^1$s is one substituent selected from the residues obtained by removing all iso(thio)nate groups from the iso(thio)cyanate compound (A).

The isocyanate compound (A) is not particularly limited, and examples thereof include hexamethylene diisocyanate, 2,2,4-trimethylhexamethylene diisocyanate, 2,4,4-trimethylhexamethylene diisocyanate, pentamethylene diisocyanate, m-xylylene diisocyanate, 1,3-tetramethylxylylene diisocyanate, isophorone diisocyanate, bis(isocyanate methyl)cyclohexane, bis(isocyanate cyclohexyl)methane, 2,5-bis(isocyanate methyl)bicyclo-[2.2.1]-heptane, 2,6-bis(isocyanate methyl)bicyclo-[2.2.1]-heptane, tolylene diisocyanate, phenylene diisocyanate, and 4,4'-diphenylmethane diisocyanate. As the isocyanate compound (A), one kind may be used, or two or more kinds may be combined.

Examples of the isothiocyanate compound (A) include aliphatic polyisothiocyanate compounds such as hexamethylene diisothiocyanate, lysine diisothiocyanate methyl ester, lysine triisothiocyanate, m-xylylene diisothiocyanate, bis(isothiocyanate methyl)sulfide, bis(isothiocyanate ethyl)sulfide, and bis(isothiocyanate ethyl)disulfide; alicyclic polyisothiocyanate compounds such as isophorone diisothiocyanate, bis(isothiocyanate methyl)cyclohexane, dicyclohexylmethane diisothiocyanate, cyclohexane diisothiocyanate, methyl cyclohexane diisothiocyanate, 2,5-bis(isothiocyanate methyl)bicyclo-[2.2.1]-heptane, 2,6-bis(isothiocyanate methyl)bicyclo-[2.2.1]-heptane, 3,8-bis(isothiocyanate methyl)tricyclodecane, 3,9-bis(isothiocyanate methyl)tricyclodecane, 4,8-bis(isothiocyanate methyl)tricyclodecane, and 4,9-bis(isothiocyanate methyl)tricyclodecane; aromatic polyisothiocyanate compounds such as tolylene diisothiocyanate, 4,4-diphenylmethane diisothiocyanate, and diphenyl sulfide-4,4-diisothiocyanate; sulfur-containing heterocyclic polyisothiocyanate compounds such as 2,5-diisothiocyanate thiophene, 2,5-bis(isothiocyanate methyl)thiophene, 2,5-isothiocyanate tetrahydrothiophene, 2,5-bis(isothiocyanate methyl)tetrahydrothiophene, 3,4-bis(isothiocyanate methyl)tetrahydrothiophene, 2,5-diisothiocyanate -1,4-dithiane, 2,5-bis(isothiocyanate methyl)-1,4-dithiane, 4,5-diisothiocyanate -1,3-dithiolane, and 4,5-bis(isothiocyanate methyl)-1,3-dithiolane. As the isothiocyanate compound (A), one kind may be used, or two or more kinds may be combined.

The iso(thio)cyanate compound (A) having two or more iso(thio)cyanate groups preferably contains at least one selected from the group consisting of m-xylylene diisocyanate, 1,3-tetramethylxylylene diisocyanate, a mixture of 2,2,4-trimethylhexamethylene diisocyanate and 2,4,4-trimethylhexamethylene diisocyanate, a mixture of 2,5-bis(isocyanatomethyl) bicyclo[2.2.1]heptane and 2,6-bis(isocyanatomethyl) bicyclo[2.2.1]heptane, and isophorone diisocyanate, and

more preferably contains at least one selected from the group consisting of m-xylylene diisocyanate, 1,3-tetramethylxylylene diisocyanate, a mixture of 2,2,4-trimethylhexamethylene diisocyanate and 2,4,4-trimethylhexamethylene diisocyanate, and a mixture of 2,5-bis(isocyanatomethyl) bicyclo[2.2.1]heptane and 2,6-bis(isocyanatomethyl) bicyclo[2.2.1] heptane.

The iso(thio)cyanate compound (A) is preferably at least one selected from the group consisting of the following

compounds (A-1) to (A-5).

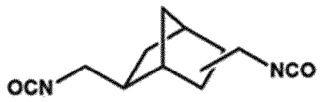

(A-1)

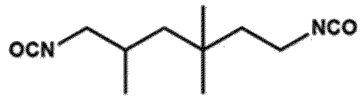

(A-2)

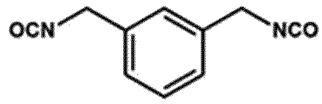

(A-3)

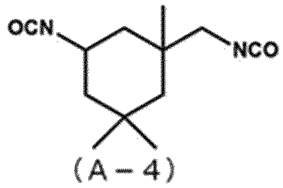

(A-4)

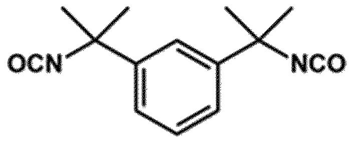

(A-5)

(Alcohol Compound (B))

The alcohol compound (B) is an alcohol compound having an allyloxy group. The alcohol compound (B) is preferably a compound having one hydroxy group and B-1 allyloxy groups in a B-valent linking group. Here, B is an integer of 2 or more and preferably from 2 to 4, and is more preferably from 2 to 4 and still more preferably 3 or 4 from the viewpoint of more excellent breaking energy and a smaller polymerization shrinkage rate when a cured product is obtained using a urethane allyl compound.

The B-valent linking group is not particularly limited, and examples thereof include groups obtained by removing B hydrogen atoms from an alkane, groups obtained by removing B hydrogen atoms from an arene, groups in which one or more of -C(=O)-, $-SO_2-$, -NR- (R represents a hydrogen atom or an alkyl group having from 1 to 10 carbon atoms, and a hydrogen atom is preferable) and the like are bonded to these groups, and groups including a combination of the above-described groups. In the group obtained by removing B hydrogen atoms from an alkane, a part of the hydrocarbon groups may be substituted with -C(=O)-, $-SO_2-$, -NR- (R represents a hydrogen atom or an alkyl group having from 1 to 10 carbon atoms, and a hydrogen atom is preferable), or the like. The B-valent linking group is preferably a group obtained by removing B hydrogen atoms from an alkane.

The number of carbon atoms in the alkane is preferably from 1 to 20, more preferably from 1 to 10, and still more preferably from 1 to 6. The alkane may have a substituent or may be unsubstituted. The alkane may be any of linear, branched, or cyclic. The cyclic alkane may be either monocyclic or polycyclic. Specific examples of the alkane include methane, ethane, propane, butane, 2-methylpropane, heptane, 2-methylbutane, and 2,2-dimethylpropane.

The number of carbon atoms in the arene is preferably from 6 to 18, more preferably from 6 to 14, and still more preferably from 6 to 10. Specific examples of the arene include benzene and condensed polycyclic aromatic compounds in which two or more aromatic rings are condensed.

In Formula (1) described above, each of $R^2$s may be independently one substituent selected from residues obtained by removing all allyloxy groups and one hydroxy group from the alcohol compound (B), and it is preferable that any of a plurality of $R^2$s is one substituent selected from residues obtained by removing all allyloxy groups and one hydroxy group from the alcohol compound (B).

The alcohol compound (B) is not particularly limited, and examples thereof include ethylene glycol monoallyl ether, trimethylolpropane diallyl ether, pentaerythritol triallyl ether, and glycerin monoallyl ether.

As the alcohol compound (B), one kind may be used, or two kinds may be combined.

The alcohol compound (B) is preferably at least one selected from the group consisting of the following compounds (B-1), (B-2), and (B-3).

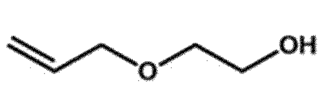
(B－1)

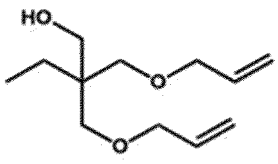
(B－2)

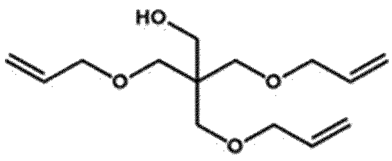
(B－3)

The urethane allyl compound of the disclosure has a molecular weight of preferably from 200 to 1500, more preferably from 300 to 1000, and still more preferably from 350 to 800.

(Method for Producing Urethane Allyl Compound)

Hereinafter, a method for producing the urethane allyl compound of the disclosure will be described. The method for producing the urethane allyl compound of the disclosure preferably includes a step of reacting the iso(thio)cyanate compound (A) with the alcohol compound (B).

In a case in which the iso(thio)cyanate compound (A) is reacted with the alcohol compound (B), a ratio ($\beta/\alpha$) of the number of moles ($\beta$) of the hydroxy group of the alcohol compound (B) to the number of moles ($\alpha$) of the iso(thio)cyanate group of the iso(thio)cyanate compound (A) is preferably from 0.5 to 1.5, more preferably from 0.8 to 1.2, and still more preferably about 1.0.

The reaction of the iso(thio)cyanate compound (A) and the alcohol compound (B) may be performed in the absence of a solvent or in a solvent. As the solvent, a known solvent can be used as long as it is a solvent inert to the reaction, and examples thereof include hydrocarbon-based solvents such as n-hexane, benzene, toluene, and xylene; ketone-based solvents such as acetone, methyl ethyl ketone, and methyl isobutyl ketone; ester-based solvents such as ethyl acetate and butyl acetate; ether-based solvents such as diethyl ether, tetrahydrofuran, and dioxane; halogen-based solvents such as dichloromethane, chloroform, carbon tetrachloride, 1,2-dichloroethane, and perchloroethylene; and polar solvents such as N,N-dimethylformamide, N,N-dimethylacetamide, N,N-dimethylimidazolidinone, dimethyl sulfoxide, and sulfolane.

These solvents may be used singly, or in combination of two or more kinds thereof.

When the iso(thio)cyanate compound (A) and the alcohol compound (B) are reacted, a catalyst may be added from the viewpoint of improving reaction rate. As the catalyst, a known catalyst that accelerates the reaction between the iso(thio)cyanate group of the iso(thio)cyanate compound (A) and the hydroxy group of the alcohol compound (B) can be used. As the catalyst, for example, a urethanization catalyst is preferably added.

Examples of the urethanization catalyst include organotin compounds such as dibutyltin dilaurate, dibutyltin dioctate, and tin octoate; organic compounds of metals other than tin such as copper naphthenate, cobalt naphthenate, zinc naphthenate, acetylacetonatozirconium, acetylacetonatoiron, and acetylacetonatogermanium; amine compounds and salts thereof such as triethylamine, 1,4-diazabicyclo[2.2.2]octane, 2,6,7-trimethyl-1-diazabicyclo[2.2.2]octane, 1,8-diazabicyclo[5.4.0]undecene, N,N-dimethylcyclohexylamine, pyridine, N-methylmorpholine, N,N,N',N'-tetramethylethylenediamine, N,N,N',N'-tetramethyl-1,3-butanediamine, N,N,N',N'-pentamethyldiethylenetriamine, N,N,N',N'-tetra(3-dimethylaminopropyl)-methanediamine, N,N'-dimethylpiperazine, and 1,2-dimethylimidazole; and trialkylphosphine compounds such as tri-n-butylphosphine, tri-n-hexylphosphine, tricyclohexylphosphine, and tri-n-octylphosphine. Among them, dibutyltin dilaurate and tin octoate, in which the reaction suitably proceeds with a small amount and the catalyst has high selectivity for the iso(thio)cyanate compound (A).

The amount of the urethanization catalyst to be used may be from 0.001% by mass to 0.1% by mass, or may be from 0.01% by mass to 0.1% by mass, with respect to the total of the iso(thio)cyanate compound (A) and the alcohol compound (B).

The reaction temperature is not particularly limited, and is usually in the range of from 20°C to 120°C, and preferably from 30°C to 100°C.

The reaction is usually carried out for a reaction time of several minutes to several tens of hours, but not particularly limited thereto, because it varies depending on conditions such as reaction temperature. The end-point of the reaction can be confirmed by a method such as HPLC (high-speed liquid chromatography) analysis.

When the iso(thio)cyanate compound (A) and the alcohol compound (B) are reacted, a polymerization inhibitor may be used from the viewpoint of suppressing a polymerization reaction of the allyloxy group in the alcohol compound (B). The polymerization inhibitor is not particularly limited, and examples thereof include dibutylhydroxytoluene (BHT), hydroquinone (HQ), hydroquinone monomethyl ether (MEHQ), and phenothiazine (PTZ).

The amount of the polymerization inhibitor to be used may be from 0.001% by mass to 0.5% by mass, may be from 0.002% by mass to 0.3% by mass, or may be from 0.005% by mass to 0.3% by mass, with respect to the total of the iso(thio)cyanate compound (A) and the alcohol compound (B).

[Monomer Composition]

The monomer composition of the disclosure contains the urethane allyl compound of the disclosure. In addition, the monomer composition of the disclosure may be for dental use, and may contain a (meth)acrylate compound (D) other than the urethane allyl compound of the disclosure.

Examples of the (meth)acrylate compound (D) include neopentyl di(meth)acrylate, 1,4-butanediol di(meth)acrylate, 1,6-hexanediol di(meth)acrylate, 1,8-octanediol di(meth)acrylate, 1,9-nonanediol di(meth)acrylate, 1,10-decanediol di(meth)acrylate, tricyclodecanedimethanol di(meth)acrylate, ethylene glycol di(meth)acrylate, diethylene glycol di(meth)acrylate, triethylene glycol di(meth)acrylate, tetraethylene glycol di(meth)acrylate, polyethylene glycol di(meth)acrylate, tripropylene glycol di(meth)acrylate, tetrapropylene glycol di(meth)acrylate, polypropylene glycol di(meth)acrylate, 2,2-bis[4-(3-(meth)acryloyloxy-2-hydroxypropoxy)phenyl]propane, ethylene oxide-modified bisphenol A di(meth)acrylate, propylene oxide-modified bisphenol A di(meth)acrylate, and 2,2,4-trimethylhexamethylene bis(2-carbamoyloxyethyl) dimethacrylate (urethane dimethacrylate: UDMA). The (meth)acrylate compound (D) may be used singly, or in combination of two or more kinds thereof. For example, a monomer for adjusting viscosity such as triethylene glycol dimethacrylate may be used in order to adjust the viscosity of the monomer composition to be low, and urethane dimethacrylate may be further used in combination in order to obtain high mechanical strength. In a case in which the monomer for adjusting viscosity and urethane dimethacrylate are used in combination, the monomer for adjusting viscosity and urethane dimethacrylate may be used at a mass ratio of from 1 : 0.8 to 1.2.

The content of the urethane allyl compound in the monomer composition is preferably from 5% by mass to 90% by mass, more preferably from 10% by mass to 70% by mass, and still more preferably from 10% by mass to 50% by mass.

In addition, the content of the (meth)acrylate compound (D) in the monomer composition is preferably from 10% by mass to 95% by mass, more preferably from 30% by mass to 90% by mass, and still more preferably from 50 to 90% by mass.

[Molded Body]

The molded body of the disclosure is a cured product of the monomer composition of the disclosure. For example, a cured product excellent in breaking strength and breaking energy can be obtained by curing a monomer composition containing the urethane allyl compound, preferably a monomer composition containing the urethane allyl compound and the (meth)acrylate compound (D).

[Composition for Dental Material]

A composition for a dental material of the disclosure contains the monomer composition of the disclosure and a polymerization initiator, and preferably further contains a filler. This composition for a dental material is autopolymerizable, thermal polymerizable or photopolymerizable, and can be preferably used, for example, as a dental restorative material. In the disclosure, a photopolymerization initiator is preferably used because a high degree of polymerization can be obtained by photopolymerization.

The amount of the monomer composition to be incorporated is preferably from 20% by mass to 80% by mass, and more preferably from 20% by mass to 50% by mass, with respect to 100% by mass of the composition for a dental material.

As the polymerization initiator, a polymerization initiator commonly used in the dental field can be used, and the polymerization initiator is selected taking into consideration the polymerizability and polymerization conditions of a polymerizable compound, such as the urethane allyl compound contained in the composition for a dental material and the (meth)acrylate compound (D).

In the case of performing autopolymerization, the polymerization initiator is preferably, for example, a redox polymerization initiator, which is a combination of an oxidizing agent and a reducing agent. In the case of using a redox polymerization initiator, the oxidizing agent and the reducing agent, which are separately packaged, may be mixed immediately before use.

The oxidizing agent is not particularly limited, and examples thereof include an organic peroxide such as a diacyl peroxide (such as benzoyl peroxide), a peroxyester (such as t-butyl peroxybenzoate), a dialkyl peroxide (such as dicumyl peroxide), a peroxyketal (such as 1,1-bis(t-butylperoxy)-3,3,5-trimethylcyclohexane), a ketone peroxide (such as methyl ethyl ketone peroxide), and a hydroperoxide (such as t-butyl hydroperoxide).

Also, the reducing agent is not particularly limited, and a tertiary amine (such as N,N-dimethylaniline) is usually used.

Besides these organic peroxide/amine systems, redox polymerization initiators such as cumene hydroperoxide/thiourea systems, ascorbic acid/$Cu^{2+}$ salt systems, and organic peroxide/amine/sulfinic acid (or salt thereof) systems

can be used. In addition, tributylborane, organic sulfinic acids, and the like are also suitably used as the polymerization initiator.

In the case of performing thermal polymerization by heating, a polymerization initiator such as a peroxide or an azo-based compound is preferred.

The peroxide is not particularly limited, and examples thereof include benzoyl peroxide, t-butyl hydroperoxide, and cumene hydroperoxide. The azo-based compound is not particularly limited, and examples thereof include azobisisobutyronitrile.

In the case of performing photopolymerization by visible light irradiation, redox initiators such as α-diketone/tertiary amine, α-diketone/aldehyde, and α-diketone/mercaptan are preferred.

The photopolymerization initiator is not particularly limited, and examples thereof include α-diketone/reducing agent, ketal/reducing agent, and thioxanthone/reducing agent.

Examples of the α-diketone include camphorquinone.

Examples of the ketal include benzyl dimethyl ketal.

Examples of the thioxanthone include 2-chlorothioxanthone.

Examples of the reducing agent include tertiary amines (such as Michler's ketone), an aldehyde (such as citronellal); and compounds having a thiol group (such as 2-mercaptobenzoxazole). A system such as α-diketone/organic peroxide/reducing agent obtained by adding an organic peroxide to these redox systems is also suitably used.

In the case of performing photopolymerization by ultraviolet irradiation, photopolymerization initiators such as benzoin alkyl ethers and benzyl dimethyl ketal are preferred. (Bis)acylphosphine oxide photopolymerization initiators are also suitably used.

Examples of the (bis)acylphosphine oxide include an acylphosphine oxide (such as 2,4,6-trimethylbenzoyldiphenylphosphine oxide) and a bisacylphosphine oxide (such as bis-(2,6-dichlorobenzoyl)phenylphosphine oxide).

These (bis)acylphosphine oxide photopolymerization initiators may be used singly or in combination with reducing agents such as various amines, aldehydes, mercaptans and sulfinates. These (bis)acylphosphine oxide photopolymerization initiators may be used in combination with the visible light photopolymerization initiators.

The polymerization initiator may be used with reference to, for example, WO 2019/107323 A and WO 2020/040141 A.

The polymerization initiator may be used singly, or in combination of two or more kinds thereof. The amount of the polymerization initiator to be incorporated is preferably from 0.01% by mass to 20% by mass, and more preferably from 0.1% by mass to 5% by mass, with respect to 100% by mass of the composition for a dental material.

As the filler, a filler commonly used in the dental field can be used. Fillers are usually classified into organic fillers and inorganic fillers

Examples of the organic filler include fine powders of polymethyl methacrylate, polyethyl methacrylate, methyl methacrylate-ethyl methacrylate copolymers, crosslinked polymethyl methacrylate, crosslinked polyethyl methacrylate, ethylene-vinyl acetate copolymers, styrene-butadiene copolymers, and the like.

Examples of the inorganic filler include fine powders of various types of glasses (which contain a silicon dioxide as a main component, and, if necessary, an oxide of a heavy metal, boron, aluminum or the like), various types of ceramics, diatomaceous earth, kaolin, clay minerals (such as montmorillonite), activated white clay, synthetic zeolite, mica, calcium fluoride, ytterbium fluoride, calcium phosphate, barium sulfate, zirconium dioxide, titanium dioxide, hydroxyapatite, and the like. Specific examples of such inorganic fillers include barium borosilicate glasses, strontium boroaluminosilicate glasses, lanthanum glasses, fluoroaluminosilicate glasses, and boroaluminosilicate glasses.

The filler may be used with reference to, for example, WO 2019/107323 A and WO 2020/040141 A.

These fillers may be used singly, or in combination of two or more kinds thereof. The amount of the filler to be incorporated may be determined as appropriate, taking into consideration handleability (viscosity) of the composition for a dental material (such as a composite resin composition), mechanical properties of a cured product thereof, and the like. The amount of the filler to be incorporated is preferably from 10 parts by mass to 2,000 parts by mass, more preferably from 50 parts by mass to 1,000 parts by mass, and still more preferably from 100 parts by mass to 600 parts by mass, with respect to 100 parts by mass of all components other than the filler contained in the composition for a dental material.

If appropriate, the composition for a dental material of the disclosure may contain a component other than the monomer composition of the disclosure, the polymerization initiator, and the filler, depending on the purpose. For example, the composition may contain the above-mentioned polymerization inhibitor for improving storage stability. Further, the composition may contain a coloring matter, such as a known pigment or dye, in order to adjust color tone. Furthermore, the composition may contain a reinforcing material such as known fibers, in order to improve strength of the cured product. In addition, the composition for a dental material of the disclosure may contain additives such as a bactericide, a disinfectant, a stabilizer, and a preservative if necessary, as long as the effects of the disclosure are achieved.

The composition for a dental material of the disclosure can be cured under appropriate conditions by the polymerization method of the polymerization initiator described above. For example, in the case of the composition for

a dental material according to the disclosure, which contains a photopolymerization initiator by visible light irradiation, a desired cured product can be obtained by processing the composition for a dental material into a predetermined shape, and then irradiating visible light for a predetermined period of time, using a known light irradiation apparatus. Conditions such as the irradiation intensity and the irradiation intensity can be appropriately changed according to curability of the composition for a dental material. In addition, the cured product which has been cured by irradiating light, such as visible light, may further be subjected to a heat treatment under appropriate conditions, to improve the mechanical properties of the cured product.

The thus obtained cured product of the composition for a dental material of the disclosure can be suitably used as a dental material.

The method of using the composition for a dental material of the disclosure is not particularly limited, as long as the method is commonly known as a method of using a dental material. For example, in the case of using the composition for a dental material of the disclosure as a composite resin for filling a caries cavity, the objective can be achieved by filling the composition for a dental material into a caries cavity in the oral cavity, and then photocuring the composition using a known light irradiation apparatus. In the case of using the composition for a dental material as a composite resin for a tooth crown, a desired tooth crown material can be obtained by processing the composition for a dental material into an appropriate shape, then photocuring the composition using a known light irradiation apparatus, and further performing a heat treatment under predetermined conditions.

The composition for a dental material and the dental material of the disclosure can be preferably used, for example, as a dental restorative material, a denture base resin, a denture base lining material, an impression material, a cementing material (such as resin cement or a resin-added glass ionomer cement), a dental adhesive (such as an orthodontic adhesive or an adhesive for cavity application), a tooth-fissure sealant, a resin block for CAD/CAM, a temporary crown, an artificial tooth material, or the like. In addition, when the dental restorative materials are classified according to application range, the dental restorative materials can be classified into composite resins for a tooth crown, composite resins for filling a caries cavity, composite resins for abutment construction, composite resins for filling restoration, and the like. Among these, the composition for a dental material and the dental material of the disclosure are particularly suitable for a dental restorative material such as a composite resin.

[Dental Material]

A dental material of the disclosure is a cured product of the composition for a dental material of the disclosure. Curing conditions for the composition for a dental material may be determined as appropriate, depending on the composition of the composition for a dental material, application of the dental material, and the like.

«Urethane Allyl (Meth)acrylate Compound»

The urethane allyl compound of the disclosure need not contain a (meth)acryloyl group, and may contain a (meth)acryloyl group.

As in the latter, in a case in which the urethane allyl compound of the disclosure contains a urethane bond, an allyloxy group, and a (meth)acryloyloxy group, the urethane allyl compound is also referred to as a urethane allyl (meth)acrylate compound of the disclosure.

The urethane allyl (meth)acrylate compound of the disclosure contains a urethane bond, an allyloxy group, and a (meth)acryloyloxy group.

The allyloxy group has higher polymerizability than the (meth)acryloyloxy group. However, in the case of using a compound containing an allyloxy group in the obtained cured product, it is difficult to obtain strength as high as in the case of using a compound containing a (meth)acryloyloxy group.

In the case of using a compound containing a (meth)acryloyloxy group, the obtained cured product is more excellent in strength than in the case of using a compound containing an allyloxy group. However, the (meth)acryloyloxy group has lower polymerizability than the allyloxy group.

It is considered that the urethane allyl (meth)acrylate compound of the disclosure can improve the strength of the allyloxy group and the polymerizability of the acryloyloxy group by containing a urethane bond, an allyloxy group, and a (meth)acryloyloxy group. As a result, the urethane allyl (meth)acrylate compound of the disclosure can obtain a cured product having excellent strength. That is, the urethane allyl (meth)acrylate compound of the disclosure can form a cured product excellent in breaking strength and breaking energy.

Furthermore, the urethane allyl (meth)acrylate compound of the disclosure can form a cured product having a low polymerization shrinkage rate.

In the urethane allyl (meth)acrylate compound of the disclosure, the number of urethane bonds, the number of allyloxy groups, and the number of (meth)acryloyloxy groups are not particularly limited. For example, the number of urethane bonds is preferably 2 or 3, and more preferably 2. The number of allyloxy groups is preferably from 2 to 9, and

more preferably from 2 to 6. The number of (meth)acryloyloxy groups is preferably from 2 to 9, and more preferably from 2 to 6.

The urethane allyl (meth)acrylate compound of the disclosure is preferably a reaction product of the iso(thio)cyanate compound (A) having two or more iso(thio)cyanate groups described above, the alcohol compound (B) having an allyloxy group described above, and an alcohol compound (C) having a (meth)acryloyloxy group (hereinafter, also referred to as "alcohol compound (C)".).

More specifically, the urethane allyl (meth)acrylate compound of the disclosure is more preferably a reaction product having a urethane bond formed by reacting an iso(thio)cyanate group of the iso(thio)cyanate compound (A) with a hydroxy group of the alcohol compound (B), and having a urethane bond formed by reacting an iso(thio)cyanate group of the iso(thio)cyanate compound (A) with a hydroxy group of the alcohol compound (C).

In addition, the urethane allyl (meth)acrylate compound of the disclosure is more preferably a reaction product having two or more urethane bonds formed by reacting two or more iso(thio)cyanate groups of the iso(thio)cyanate compound (A) with a hydroxy group of the alcohol compound (B) or a hydroxy group of the alcohol compound (C).

The urethane allyl (meth)acrylate compound of the disclosure is preferably a compound represented by the following Formula (Y1).

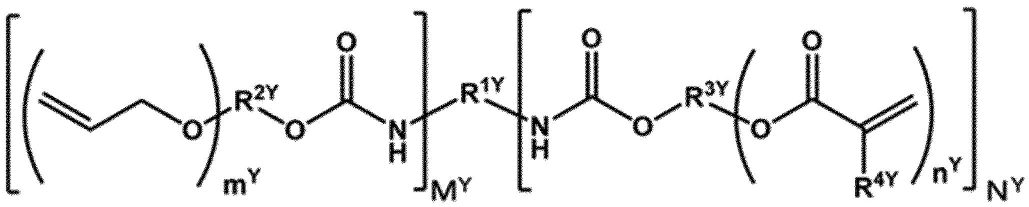

(Y1)

wherein $R^{1Y}$ is a residue obtained by removing $M^Y + N^Y$ iso(thio)cyanate groups from the iso(thio)cyanate compound (A) having two or more iso(thio)cyanate groups.

$R^{2Y}$ is a residue obtained by removing $m^Y$ allyloxy groups and one hydroxy group from the alcohol compound (B) having an allyloxy group.

$R^{3Y}$ is a residue obtained by removing $n^Y$ (meth)acryloyloxy groups and one hydroxy group from the alcohol compound (C) having a (meth)acryloyloxy group.

$R^{4Y}$ is a hydrogen atom or a methyl group. $n^Y$ and $m^Y$ are integers of 1 or more, $M^Y$ is an integer from 1 to 3, $N^Y$ is an integer from 1 to 3, and $M^Y + N^Y$ is an integer from 2 to 4.

In a case in which there is a plurality of $R^{2Y}$s, $R^{3Y}$s, or $R^{4Y}$s, the plurality of $R^{2Y}$s, $R^{3Y}$s, or $R^{4Y}$s may be the same or different.

It is more preferable that $m^Y$ is from 1 to 3 from the viewpoint of more excellent breaking energy and a smaller polymerization shrinkage rate of the obtained cured product.

$M^Y$ is an integer from 1 to 3, and more preferably 1.

$n^Y$ is preferably from 1 to 3, more preferably 1 or 2, and still more preferably 1 from the viewpoint of more excellent breaking energy and a smaller polymerization shrinkage rate of the obtained cured product.

$N^Y$ is an integer from 1 to 3, and more preferably 1.

Hereinafter, an iso(thio)cyanate compound (A) having two or more iso(thio)cyanate groups, an alcohol compound (B) having an allyloxy group and an alcohol compound (C) having a (meth)acryloyloxy group, which can be used for producing the urethane allyl (meth)acrylate compound of the disclosure, and reaction conditions thereof will be described.

(Iso(thio)cyanate Compound (A))

Since the urethane allyl (meth)acrylate compound of the disclosure is one aspect of the urethane allyl compound of the disclosure, the iso(thio)cyanate compound (A) described in the above description of the urethane allyl compound can be used.

(Alcohol Compound (B))

Since the urethane allyl (meth)acrylate compound of the disclosure is one aspect of the urethane allyl compound

of the disclosure, the alcohol compound (B) described in the above description of the urethane allyl compound can be used.

(Alcohol Compound (C))

The alcohol compound (C) is an alcohol compound having a (meth)acryloyloxy group. The alcohol compound (C) is preferably a compound having one hydroxy group and C-1 (meth)acryloyloxy groups in the C-valent linking group. Here, C is an integer of 2 or more, preferably from 2 to 4, more preferably 2 or 3, and still more preferably 2 from the viewpoint of more excellent breaking energy and a smaller polymerization shrinkage rate of the obtained cured product.

As the alcohol compound (C), one kind may be used, or two kinds may be combined.

The C-valent linking group is not particularly limited, and is preferably, for example, a linear or branched divalent acyclic hydrocarbon group having from 1 to 50 carbon atoms, a divalent cyclic hydrocarbon group having from 3 to 50 carbon atoms, or a divalent organic group having from 1 to 50 carbon atoms and containing an oxygen atom in a main chain. The divalent cyclic hydrocarbon group having from 3 to 50 carbon atoms may be composed of only a cyclic hydrocarbon moiety, or may be a combination of a cyclic hydrocarbon moiety and an acyclic hydrocarbon moiety.

Here, examples of the divalent acyclic hydrocarbon group include alkylene groups, alkenylene groups, and alkynylene groups, and examples of the divalent cyclic hydrocarbon group include cycloalkylene groups, cycloalkenylene groups, cycloalkynylene groups, and arylene groups. In addition, the divalent organic group containing an oxygen atom in the main chain preferably does not have a structure in which oxygen atoms are continuously present in the main chain, for example, "-O-O-", and the structure other than the oxygen atoms is preferably a hydrocarbon group. Moreover, a hydrogen atom in the acyclic hydrocarbon group and the hydrocarbon group may be substituted with a halogen atom such as a chlorine atom or a bromine atom, or a substituent such as an alkoxy group, a nitro group, a hydroxy group, or a carbonyl group, and a hydrogen atom in the cyclic hydrocarbon group may be substituted with a halogen atom such as a chlorine atom or a bromine atom, or a substituent such as an alkoxy group, a nitro group, a hydroxy group, a carbonyl group, or an alkyl group.

As the linear or branched divalent acyclic hydrocarbon group having from 1 to 50 carbon atoms, a linear or branched divalent acyclic hydrocarbon group having from 1 to 20 carbon atoms is preferred, and a linear or branched divalent acyclic hydrocarbon group having from 1 to 10 carbon atoms is more preferred.

The divalent cyclic hydrocarbon group having from 3 to 50 carbon atoms is preferably a divalent cyclic hydrocarbon group having from 6 to 30 carbon atoms, and more preferably a divalent cyclic hydrocarbon group having from 10 to 25 carbon atoms.

As the divalent organic group having from 1 to 50 carbon atoms and containing an oxygen atom in the main chain, an oxyalkylene group having from 1 to 50 carbon atoms is preferred, and an oxyalkylene group having from 2 to 30 carbon atoms is more preferred.

In Formula (1) described above, each of $R^3$s may be independently one substituent selected from residues obtained by removing all (meth)acryloyloxy groups and one hydroxy group from the alcohol compound (C), and it is preferable that any of a plurality of $R^3$s is one substituent selected from residues obtained by removing all (meth)acryloyloxy groups and one hydroxy group from the alcohol compound (C).

The alcohol compound (C) is not particularly limited, and examples thereof include 2-hydroxyethyl acrylate (HEA), 2-hydroxypropyl acrylate (HPA), 2-hydroxyethyl methacrylate (HEMA), 2-hydroxypropyl methacrylate (HPMA), 2-hydroxybutyl (meth)acrylate, 4-hydroxybutyl (meth)acrylate (4HBA), 2-hydroxy-3-phenoxypropyl acrylate, and 1,4-cyclohexanedimethanol monoacrylate.

The alcohol compound (C) having a (meth)acryloyloxy group preferably contains at least one selected from the group consisting of 2-hydroxyethyl (meth)acrylate, 2-hydroxypropyl (meth)acrylate, 2-hydroxybutyl (meth)acrylate, 4-hydroxybutyl (meth)acrylate (4HBA), 2-hydroxy-3-phenoxypropyl (meth)acrylate, and 1,4-cyclohexanedimethanol mono(meth)acrylate.

The molecular weight of the urethane allyl (meth)acrylate compound of the disclosure is preferably from 200 to 1500, more preferably from 300 to 1000, and still more preferably from 350 to 800.

(Method for Producing Urethane Allyl (Meth)acrylate Compound)

Hereinafter, a method for producing the urethane allyl (meth)acrylate compound of the disclosure will be described. The method for producing a urethane allyl (meth)acrylate compound of the disclosure preferably includes a step of reacting the iso(thio)cyanate compound (A), the alcohol compound (B), and the alcohol compound (C).

In the above step, the iso(thio)cyanate compound (A), the alcohol compound (B), and the alcohol compound (C) may be reacted at a time, the alcohol compound (C) may be reacted after reacting the iso(thio)cyanate compound (A) and the alcohol compound (B), or the alcohol compound (B) may be reacted after reacting the iso(thio)cyanate compound (A) and the alcohol compound (C).

From the viewpoint of efficiently producing the urethane allyl (meth)acrylate compound of the disclosure, it is

preferable to react the iso(thio)cyanate compound (A), the alcohol compound (B), and the alcohol compound (C) at a time in the above step.

When the iso(thio)cyanate compound (A) is reacted with the alcohol compound (C), a ratio ($\gamma/\alpha$) of the number of moles ($\gamma$) of the hydroxy group of the alcohol compound (C) to the number of moles ($\alpha$) of the iso(thio)cyanate group of the iso(thio)cyanate compound (A) is preferably from 0.5 to 1.5, more preferably from 0.8 to 1.2, and still more preferably about 1.0.

The reaction of the iso(thio)cyanate compound (A), the alcohol compound (B), and the alcohol compound (C) may be performed in the absence of a solvent or in a solvent. As the solvent, a known solvent can be used as long as it is a solvent inert to the reaction, and examples thereof include the above-described solvents.

These solvents may be used singly, or in combination of two or more kinds thereof.

(Catalyst)

When the iso(thio)cyanate compound (A), the alcohol compound (B), and the alcohol compound (C) are reacted, a catalyst may be added from the viewpoint of improving reaction rate. As the catalyst, a known catalyst that accelerates a reaction between an iso(thio)cyanate group of the iso(thio)cyanate compound (A) and a hydroxy group of the alcohol compound (B) or a reaction between an iso(thio)cyanate group of the iso(thio)cyanate compound (A) and a hydroxy group of the alcohol compound (C) can be used. As the catalyst, for example, a urethanization catalyst is preferably added.

Examples of the urethanization catalyst include the above-described urethanization catalysts, and preferred aspects are also the same.

The amount of the urethanization catalyst to be used may be from 0.001% by mass to 0.1% by mass, or may be from 0.01% by mass to 0.1% by mass, with respect to the total of the iso(thio)cyanate compound (A), the alcohol compound (B), and the alcohol compound (C).

The reaction temperature is not particularly limited, and is usually in the range of from 20°C to 120°C, and preferably from 30°C to 100°C.

The reaction is usually carried out for a reaction time of several minutes to several tens of hours, but not particularly limited thereto, because it varies depending on conditions such as reaction temperature. The end-point of the reaction can be confirmed by a method such as HPLC (high-speed liquid chromatography) analysis.

When the iso(thio)cyanate compound (A), the alcohol compound (B), and the alcohol compound (C) are reacted, a polymerization inhibitor may be used from the viewpoint of suppressing the polymerization reaction of the allyloxy group in the alcohol compound (B), and a polymerization reaction of the acryloyloxy group in the alcohol compound (C). Examples of the polymerization inhibitor include the above-described polymerization inhibitors.

The amount of the polymerization inhibitor to be used may be from 0.001% by mass to 0.5% by mass, may be from 0.002% by mass to 0.3% by mass, or may be from 0.005% by mass to 0.3% by mass, with respect to the total of the iso(thio)cyanate compound (A), the alcohol compound (B), and the alcohol compound (C).

<Monomer Composition B>

In the monomer composition of the disclosure, the urethane allyl compound may be a urethane allyl (meth)acrylate compound. The monomer composition in this case is referred to as a monomer composition B.

The monomer composition B of the disclosure may also be for dental use.

By containing the urethane allyl (meth)acrylate compound of the disclosure, the monomer composition B of the disclosure can impart a function such as mechanical strength to, for example, the obtained cured product using the monomer composition B of the disclosure.

The monomer composition B of the disclosure may contain the urethane allyl (meth)acrylate compound of the disclosure and a (meth)acrylate compound other than the urethane allyl (meth)acrylate compound of the disclosure.

Examples of the (meth)acrylate compound other than the urethane allyl (meth)acrylate compound of the disclosure include a (meth)acrylate compound intended to lower the viscosity of the monomer composition B of the disclosure (for example, the (meth)acrylate compound (D) described later), and a (meth)acrylate compound intended to improve the strength of the cured product (for example, the (meth)acrylate compound (E) described later).

The monomer composition B of the disclosure may contain a (meth)acrylate compound (D) other than the urethane allyl (meth)acrylate compound of the disclosure.

In general, in a case in which a main component monomer capable of improving the strength of the cured product is selected, viscosity of the monomer composition B increases, and handleability may be deteriorated.

Therefore, from the viewpoint of reducing the viscosity of the monomer composition B and improving the handleability, a diluent monomer may be added to the main component monomer. Examples of the conventionally known diluent monomer include (meth)acrylates such as triethylene glycol dimethacrylate.

However, in a case in which the above-described conventionally known diluent monomer is added to the main

component monomer to form the monomer composition B, functions such as toughness in the cured product may be deteriorated. On the other hand, in the case of using the (meth)acrylate compound (D) of the disclosure as a diluent monomer for improving the handleability of the monomer composition B, the mechanical properties of the cured product tend to be improved as compared with the case of using the above-described conventionally known diluent monomer.

Therefore, in the monomer composition B of the disclosure, for example, a main component monomer polymerizable in the monomer composition B, the urethane allyl (meth)acrylate compound of the disclosure, and the (meth)acrylate compound (D) in the disclosure as a diluent monomer may be used in combination.

Examples of the (meth)acrylate compound (D) include the (meth)acrylate compound (D) described in the description of the monomer composition of the disclosure.

A preferred aspect of the (meth)acrylate compound (D) is as described in the section of the (meth)acrylate compound (D) described above.

The monomer composition B of the disclosure may contain a (meth)acrylate compound (E) other than the urethane allyl (meth)acrylate compound of the disclosure.

The monomer composition B of the disclosure can improve the strength of the cured product by containing the (meth)acrylate compound (E).

Examples of the (meth)acrylate compound (E) include ethylene oxide-modified bisphenol A di(meth)acrylate, propylene oxide-modified bisphenol A di(meth)acrylate, and 2,2,4-trimethylhexamethylene bis(2-carbamoyloxyethyl) dimethacrylate (urethane dimethacrylate: UDMA). In addition, the (meth)acrylate compound (E) may be a reaction product of (i) a thiol compound having three or more thiol groups, (ii) an iso(thio)cyanate compound having two or more iso(thio)cyanate groups, and (iii) a (meth)acrylate compound having a (meth)acryloyloxy group and having a hydroxy group.

<Molded Body B>

The molded body of the disclosure may be a cured product of the monomer composition B as the monomer composition of the disclosure. The molded body in this case is referred to as a molded body B.

For example, a cured product B excellent in breaking strength and breaking energy can be obtained by curing a monomer composition containing the urethane allyl (meth)acrylate compound, preferably a monomer composition containing the urethane allyl (meth)acrylate compound and the (meth)acrylate compound (D).

<Composition for Dental Material B>

The composition for a dental material of the disclosure may contain the monomer composition B as the monomer composition of the disclosure and a polymerization initiator. The composition for a dental material in this case is referred to as a composition for a dental material B.

The composition for a dental material B of the disclosure preferably further contains a filler. This composition for a dental material B is autopolymerizable, thermal polymerizable or photopolymerizable, and can be preferably used, for example, as a dental restorative material. In the disclosure, a photopolymerization initiator is preferably used because a high degree of polymerization can be obtained by photopolymerization.

Since the composition for a dental material B is one aspect of the composition for a dental material described above, the amount of the monomer composition to be incorporated in the composition for a dental material B is the same as the amount to be incorporated described above.

Since the composition for a dental material B is one aspect of the composition for a dental material described above, the polymerization initiator in the composition for a dental material B is as described in the section of the polymerization initiator described above.

Details of the filler are as described in the section of the filler described above.

If appropriate, the composition for a dental material B of the disclosure may contain a component other than the monomer composition B of the disclosure, the polymerization initiator, and the filler, depending on the purpose. In addition, the composition for a dental material B of the disclosure may contain other additives.

Details are as described above.

The composition for a dental material B of the disclosure can be cured under appropriate conditions by the polymerization method of the polymerization initiator described above. Details are as described above.

The thus obtained cured product of the composition for a dental material B of the disclosure can be suitably used as a dental material B. Details are as described above.

The composition for a dental material B and the dental material B of the disclosure can be used for the above-described applications.

<Dental Material B>

The dental material B of the disclosure is a cured product of the composition for a dental material B of the disclosure. Curing conditions for the composition for a dental material B may be determined as appropriate, depending on the composition of the composition for a dental material B, application of the dental material B, and the like.

[Examples]

Hereinafter, the disclosure will be described more specifically with reference to examples, but the disclosure is not limited by these examples.
Abbreviations of compounds used in the Examples of the disclosure are shown below.

EGMA: ethylene glycol monoallyl ether
TMPDA: trimethylolpropane diallyl ether
PETA: pentaerythritol triallyl ether
TMHDI: a mixture of 2,2,4-trimethylhexamethylene diisocyanate and 2,4,4-trimethylhexamethylene diisocyanate
NBDI: a mixture of 2,5-bis(isocyanatomethyl) bicyclo[2.2.1]heptane and 2,6-bis(isocyanatomethyl) bicyclo[2.2.1] heptane
XDI: m-xylylene diisocyanate
IPDI: isophorone diisocyanate
TMXDI: 1,3-tetramethylxylylene diisocyanate
DBTDL: dibutyltin dilaurate
BHT: dibutylhydroxytoluene
CQ: camphorquinone
DMAB2-BE: 2-butoxyethyl 4-dimethylaminobenzoate
UDMA: 2,2,4-trimethylhexamethylene bis(2-carbamoyloxyethyl) dimethacrylate DAP: diallyl phthalate
BAC: diethylene glycol bisallyl carbonate
HEA: 2-hydroxyethyl acrylate
HEMA: 2-hydroxyethyl methacrylate
HPA: 2-hydroxypropyl acrylate
HPMA: 2-hydroxypropyl methacrylate
4HBA: 4-hydroxybutyl acrylate
3G: triethylene glycol dimethacrylate

[Method for Measuring IR Spectrum]

An IR spectrum of the urethane allyl compound obtained in each Example was measured using a Fourier transform infrared spectroscopic analyzer, Spectrum Two/UATR (Universal Attenuated Total Reflectance) manufactured by PerkinElmer Japan Co., Ltd.
The urethane allyl compound obtained in each Example was allowed to stand at 20°C for 24 hours, and an infrared absorption spectrum was then measured for the urethane allyl compound at 20°C.

[Bending Test Method]

A bending test method in Examples and Comparative Examples of the disclosure will be shown below.

(Fabrication of Bending Test Pieces)

0.05 Parts by mass of CQ and 0.05 parts by mass of DMAB2-BE were added into 10 parts by mass of the monomer composition obtained in each of Examples and Comparative Examples, and the mixture was stirred to uniformity at room temperature. Further, 15 parts by mass of silica glass (Fuselex-X (TATSUMORI LTD.)) was added to the mixture, and then the mixture was stirred to uniformity using a mortar, followed by defoaming to prepare a composition for a dental material. The obtained composition for a dental material was put into a stainless steel mold of 2 mm × 2 mm × 25 mm, and irradiated with light using a visible light irradiator (Solidilite V manufactured by SHOFU INC.) for 3 minutes on each side, for a total of 6 minutes on both sides, to obtain a cured product. Further, the cured product removed from the mold was heat treated in an oven at 130°C for 2 hours. The cured product removed from the oven was cooled to room temperature, then the cured product was soaked in distilled water in a sealable sample bottle, and held at 37°C for 24 hours, and the resulting product was used as a test piece (bending test piece).

(Bending Test)

The test pieces fabricated in the above method were subjected to a three-point bending test using a tester (AUTOGRAPH EZ-S manufactured by Shimadzu Corporation) at a distance between supporting points of 20 mm and a crosshead speed of 1 mm/min to measure breaking strength and breaking energy.

[Method of Measuring Polymerization Shrinkage Rate]

A method of measuring polymerization shrinkage rate in Examples and Comparative Examples of the disclosure will be shown below.

(Preparation of Sample for Measuring Polymerization Shrinkage Rate)

0.1 Parts by mass of CQ and 0.1 parts by mass of DMAB2-BE were added to 20 parts by mass of the monomer composition obtained in each of Examples and Comparative Examples, and the mixture was stirred and dissolved to uniformity at room temperature, then a composition for measuring polymerization shrinkage rate was prepared. The obtained composition for measuring polymerization shrinkage rate was filled into a silicon mold having a diameter of 10 mm and a depth of 2 mm, sandwiched from above and below with a cover glass, and then irradiated with light using a visible light irradiator (Solidilite V manufactured by SHOFU INC.) for 3 minutes on each side, for a total of 6 minutes on both sides. A test piece removed from the mold whose surface was wiped with acetone was used as a test sample (sample for measuring polymerization shrinkage rate).

(Measurement of Polymerization Shrinkage Rate)

Density of the monomer composition before and after curing was measured using a dry densitometer (AccuPyc 1330 manufactured by Shimadzu Corporation), and the polymerization shrinkage rate was determined from the following Equation (1).

$$\text{Equation (1): Polymerization shrinkage rate (\%)} = ((\text{Density after polymerization} - \text{Density before Polymerization})/\text{Density after polymerization}) \times 100$$

Subsequently, the polymerization shrinkage rate of UDMA alone was measured by the same operation as described above. As a result, the polymerization shrinkage rate was 7.5%.

Subsequently, using polymerization shrinkage rates of the monomer compositions obtained in each of Examples and Comparative Examples (S1) and polymerization shrinkage rate of UDMA alone (S2, 7.5%), polymerization shrinkage rates of urethane allyl compounds (A-1) to (A-7) and allyl compounds (DAP and BAC) (S3) were determined from the following Equation (2).

$$\text{Equation (2): } (S3) = ((S1) - ((S2) \times 0.8))/0.2$$

[Method of Measuring Refractive Index]

In Examples and Comparative Examples of the disclosure, the refractive index was measured using an Abbe type full digital refractive index system (Abbemat 550 manufactured by Anton Paar GmbH). The temperature was controlled at 25°C.

[Example 1A]

0.05 Parts by mass of DBTDL, 0.025 parts by mass of BHT, and 24.64 parts by mass of TMHDI were charged into a 100-mL four-necked flask equipped with a sufficiently dried stirring blade and a thermometer, and dissolved to obtain a homogeneous solution. Then, the solution was heated to 80°C, and 25.36 parts by mass of EGMA was further added dropwise to the solution over 1 hour. Since the internal temperature increased due to reaction heat during the dropwise addition, the amount of dropwise addition was controlled so as to be 90°C or less. After the whole amount of EGMA was added dropwise, the reaction was performed for 5 hours while maintaining the reaction temperature at 90°C. At this time, progress of the reaction was followed by HPLC analysis to confirm end point of the reaction. The product

was discharged from the reactor to obtain 50 g of a urethane allyl compound (A-1). The urethane allyl compound (A-1) had a refractive index of 1.4793 at 25°C. An IR spectrum of the urethane allyl compound (A-1) is shown in Fig. 1A. 3.0 Parts by mass of the obtained urethane allyl compound (A-1) and 12.0 parts by mass of UDMA were put in a container, and the mixture was stirred to uniformity at 50°C to obtain a monomer composition (1A). A composition for a dental material (1A) and a test piece (bending test piece) were obtained from the obtained monomer composition (1A) according to the methods described in the sections of (Fabrication of Bending Test Pieces) and (Bending Test). The test piece was subjected to bending test, and was found to have a breaking strength of 181 MPa and a breaking energy of 54 mJ. A composition for measuring polymerization shrinkage rate (1A) and a test sample (sample for measuring polymerization shrinkage rate) were obtained from the obtained monomer composition (1A) according to the methods described in the sections of (Preparation of Sample for Measuring Polymerization Shrinkage Rate) and (Measurement of Polymerization Shrinkage Rate). The test sample was subjected to measurement of polymerization shrinkage rate, and was found to have a polymerization shrinkage rate of 4.50%.

[Examples 2A to 11A]

Urethane allyl compounds (A-2) to (A-11) were obtained in the same manner as in Example 1A except that the alcohol compound (B) and the isocyanate compound (A) were changed to compounds shown in Table 1. Refractive indexes of the urethane allyl compounds (A-2) to (A-11) at 25°C are as shown in Table 1. IR spectra of the urethane allyl compounds (A-2) to (A-11) are shown in Figs. 2A to 11A. In addition, monomer compositions (2A) to (11A) were each obtained in the same manner as in Example 1A except that the urethane allyl compound (A-1) was changed to each of the urethane allyl compounds (A-2) to (A-11). Compositions for a dental material (2A) to (11A) and test pieces (bending test pieces) were obtained from the obtained monomer compositions (2A) to (11A) according to the methods described in the sections of (Fabrication of Bending Test Pieces) and (Bending Test), and the test pieces were subjected to bending test. Breaking strength and breaking energy are shown in Table 1. Further, compositions for measuring polymerization shrinkage rate (2) to (11) and test samples (samples for measuring polymerization shrinkage rate) were obtained from the obtained monomer compositions (2A) to (11A) according to the methods described in the sections of (Preparation of Sample for Measuring Polymerization Shrinkage Rate) and (Measurement of Polymerization Shrinkage Rate), and the test samples were subjected to measurement of polymerization shrinkage rate. The polymerization shrinkage rates are shown in Table 1.

[Comparative Example 1A]

3.0 Parts by mass of DAP and 12.0 parts by mass of UDMA were put in a container, and the mixture was stirred to uniformity at 50°C to obtain a monomer composition (12A). A composition for a dental material (12A) and a test piece (bending test piece) were obtained from the obtained monomer composition (12A) according to the methods described in the sections of (Fabrication of Bending Test Pieces) and (Bending Test). The test piece was subjected to bending test, and was found to have a breaking strength of 133 MPa, and a breaking energy of 12 mJ. A composition for measuring polymerization shrinkage rate (12A) and a test sample (sample for measuring polymerization shrinkage rate) were obtained from the obtained monomer composition (12A) according to the methods described in the sections of (Preparation of Sample for Measuring Polymerization Shrinkage Rate) and (Measurement of Polymerization Shrinkage Rate). The test sample was subjected to measurement of polymerization shrinkage rate, and was found to have a polymerization shrinkage rate of 14.00%.

[Comparative Example 2A]

3.0 Parts by mass of BAC and 12.0 parts by mass of UDMA were put in a container, and the mixture was stirred to uniformity at 50°C to obtain a monomer composition (13A). A composition for a dental material (13A) and a test piece (bending test piece) were obtained from the obtained monomer composition (13A) according to the methods described in the sections of (Fabrication of Bending Test Pieces) and (Bending Test). The test piece was subjected to bending test, and was found to have a breaking strength of 119 MPa, and a breaking energy of 10 mJ. A composition for measuring polymerization shrinkage rate (13A) and a test sample (sample for measuring polymerization shrinkage rate) were obtained from the obtained monomer composition (13A) according to the methods described in the sections of (Preparation of Sample for Measuring Polymerization Shrinkage Rate) and (Measurement of Polymerization Shrinkage Rate). The test sample was subjected to measurement of polymerization shrinkage rate, and was found to have a polymerization shrinkage rate of 13.50%.

[Table 1]

| | | Charging ratio for production of urethane allyl compound | | Monomer physical properties | | Cured product physical properties | |
|---|---|---|---|---|---|---|---|
| | | Alcohol compound (parts by mass) | Isocyanate compound (parts by mass) | Refractive index | Polymerization shrinkage rate [%] | Breaking strength [MPa] | Breaking energy [mJ] |
| Examples | 1A | EGMA 25.36 | TMHDI 24.64 | 1.4793 | 4.50 | 181 | 54 |
| | 2A | EGMA 25.12 | NBDI 24.88 | 1.4992 | 4.46 | 194 | 34 |
| | 3A | EGMA 23.97 | XDI 26.03 | 1.5192 | 5.95 | 182 | 39 |
| | 4A | EGMA 26.05 | IPDI 23.95 | 1.4912 | 3.45 | 194 | 35 |
| | 5A | EGMA 27.23 | TMXDI 22.77 | 1.5107 | 3.52 | 188 | 37 |
| | 6A | TMPDA 34.75 | XDI 15.25 | 1.5076 | 3.50 | 188 | 52 |
| Alcohol compound (parts by mass) | Isocyanate compound (parts by mass) | Refractive index | Alcohol compound (parts by mass) | Isocyanate compound (parts by mass) | Refractive index | | |
| Examples | 7A | PETA 36.58 | XDI 13.42 | 1.5060 | 3.49 | 186 | 50 |
| | 8A | TMPDA 31.85 | TMHDI 18.15 | 1.4802 | 4.30 | 175 | 42 |
| | 9A | PETA 33.87 | TMHDI 16.13 | 1.4817 | 4.43 | 171 | 44 |
| | 10A | TMPDA 33.76 | NBDI 16.24 | 1.4930 | 4.27 | 181 | 53 |
| | 11A | PETA 35.66 | NBDI 14.34 | 1.4929 | 4.21 | 181 | 50 |
| Comparative Examples | 1A | - | - | 1.5190 | 14.00 | 133 | 12 |
| | 2A | - | - | 1.4261 | 13.50 | 119 | 10 |

As shown in Table 1, the monomer compositions of Examples 1A to 11A could reduce the polymerization shrinkage rate and were excellent in breaking strength and breaking energy as compared with the monomer compositions of Comparative Examples 1A and 2A.

(Example 1B)

0.05 Parts by mass of DBTDL, 0.025 parts by mass of BHT, and 23.15 parts by mass of XDI were charged into a 100-mL four-necked flask equipped with a sufficiently dried stirring blade and a thermometer, and dissolved to obtain a homogeneous solution. Then, the solution was heated to 80°C, and 12.57 parts by mass of EGMA and 14.28 parts by mass of HEA were further added dropwise to the solution over 1 hour. Since the internal temperature increased due to reaction heat during the dropwise addition, the amount of dropwise addition was controlled so as to be 90°C or less. After the whole amount of EGMA was added dropwise, the reaction was performed for 5 hours while maintaining the reaction temperature at 90°C. At this time, progress of the reaction was followed by HPLC analysis to confirm end point of the reaction. The product was discharged from the reactor to obtain 50 g of a urethane allyl (meth)acrylate compound (B-1).

The urethane allyl (meth)acrylate compound (B-1) had a refractive index of 1.4793 at 25°C. An IR spectrum of the urethane allyl (meth)acrylate compound (B-1) is shown in Fig. 1B.

3.0 Parts by mass of the obtained urethane allyl (meth)acrylate compound (B-1) and 12.0 parts by mass of UDMA were put in a container, and the mixture was stirred to uniformity at 50°C to obtain a monomer composition (1).

A composition for a dental material (1B) and a test piece (bending test piece) were obtained from the obtained monomer composition (1B) according to the methods described in the sections of (Fabrication of Bending Test Pieces) and (Bending Test), and the test piece was subjected to bending test. Breaking strength and breaking energy are shown in Table 2.

A composition for measuring polymerization shrinkage rate (1B) and a test sample (sample for measuring polymerization shrinkage rate) were obtained from the obtained monomer composition (1B) according to the methods described in the sections of (Preparation of Sample for Measuring Polymerization Shrinkage Rate) and (Measurement of Polymerization Shrinkage Rate), and the test sample was subjected to measurement of polymerization shrinkage rate. The results are shown in Table 2.

(Example 2B to Example 13B)

Urethane allyl (meth)acrylate compounds (B-2) to (B-13) were obtained in the same manner as in Example 1B except that the alcohol compounds (B) and (C) and the isocyanate compound (A) were changed to compounds shown in Table 2. Refractive indexes of the compounds at 25°C are as shown in Table 2. IR spectra of the urethane allyl (meth)acrylate compounds (B-2) to (B-13) are shown in Figs. 2B to 13B. In addition, monomer compositions (2B) to (13B) were each obtained in the same manner as in Example 1B except that the urethane allyl (meth)acrylate compound (B-1) was changed to each of the urethane allyl (meth)acrylate compounds (B-2) to (B-13). Compositions for a dental material (2) to (13B) and test pieces (bending test pieces) were obtained from the obtained monomer compositions (2B) to (13B) according to the methods described in the sections of (Fabrication of Bending Test Pieces) and (Bending Test), and the test pieces were subjected to bending test. Breaking strength and breaking energy are shown in Table 2. Further, compositions for measuring polymerization shrinkage rate (2B) to (13B) and test samples (sample for measuring polymerization shrinkage rate) were obtained from the obtained monomer compositions (2B) to (13B) according to the methods described in the sections of (Preparation of Sample for Measuring Polymerization Shrinkage Rate) and (Measurement of Polymerization Shrinkage Rate), and the test samples were subjected to measurement of polymerization shrinkage rate. The polymerization shrinkage rates are shown in Table 2.

(Comparative Example 1B)

3.0 Parts by mass of DAP and 12.0 parts by mass of UDMA were put in a container, and the mixture was stirred to uniformity at 50°C to obtain a monomer composition (14B). A composition for a dental material (14B) and a test piece (bending test piece) were obtained from the obtained monomer composition (14B) according to the methods described in the sections of (Fabrication of Bending Test Pieces) and (Bending Test), and the test piece was subjected to bending test. Breaking strength and breaking energy are shown in Table 2.

A composition for measuring polymerization shrinkage rate (14B) and a test sample (sample for measuring polymerization shrinkage rate) were obtained from the obtained monomer composition (14B) according to the methods described in the sections of (Preparation of Sample for Measuring Polymerization Shrinkage Rate) and (Measurement of Polymerization Shrinkage Rate), and the test sample was subjected to measurement of polymerization shrinkage rate. The results are shown in Table 2.

A refractive index of DAP is shown in Table 2.

(Comparative Example 2B)

3.0 Parts by mass of BAC and 12.0 parts by mass of UDMA were put in a container, and the mixture was stirred to uniformity at 50°C to obtain a monomer composition (15B). A composition for a dental material (15B) and a test piece (bending test piece) were obtained from the obtained monomer composition (15B) according to the methods described in the sections of (Fabrication of Bending Test Pieces) and (Bending Test), and the test piece was subjected to bending test. Breaking strength and breaking energy are shown in Table 2.

A composition for measuring polymerization shrinkage rate (15B) and a test sample (sample for measuring polymerization shrinkage rate) were obtained from the obtained monomer composition (15B) according to the methods described in the sections of (Preparation of Sample for Measuring Polymerization Shrinkage Rate) and (Measurement of Polymerization Shrinkage Rate), and the test sample was subjected to measurement of polymerization shrinkage rate. The results are shown in Table 2.

A refractive index of BAC is shown in Table 2.

(Comparative Example 3B)

3.0 Parts by mass of 3G and 12.0 parts by mass of UDMA were put in a container, and the mixture was stirred to uniformity at 50°C to obtain a monomer composition (16B). A composition for a dental material (16B) and a test piece (bending test piece) were obtained from the obtained monomer composition (16B) according to the methods described in the sections of (Fabrication of Bending Test Pieces) and (Bending Test), and the test piece was subjected to bending test. Breaking strength and breaking energy are shown in Table 2.

A composition for measuring polymerization shrinkage rate (16B) and a test sample (sample for measuring polymerization shrinkage rate) were obtained from the obtained monomer composition (16B) according to the methods described in the sections of (Preparation of Sample for Measuring Polymerization Shrinkage Rate) and (Measurement of Polymerization Shrinkage Rate), and the test sample was subjected to measurement of polymerization shrinkage rate. The results are shown in Table 2.

A refractive index of 3G is shown in Table 2.

[Table 2]

| | | Charging ratio | | | Monomer physical properties | | Evaluation | |
|---|---|---|---|---|---|---|---|---|
| | | Alcohol compound (B) (pbW) | Alcohol compound (C) (pbW) | Isocyanate compound (A) (pbW) | Refractive index | Polymerization shrinkage rate [%] | Breaking strength [MPa] | Breaking energy [mJ] |
| Examples | 1B | EGMA 12.57 | HEA 14.28 | XDI 23.15 | Solid | 5.50 | 187 | 38 |
| | 2B | EGMA 12.15 | HEMA 15.47 | XDI 22.38 | Solid | 6.50 | 195 | 38 |
| | 3B | EGMA 12.15 | HPA 15.47 | XDI 22.38 | 1.5176 | 6.00 | 210 | 43 |
| | 4B | EGMA 11.76 | HPMA 16.59 | XDI 21.65 | 1.5144 | 6.00 | 204 | 38 |
| | 5B | EGMA 11.92 | HEA 13.55 | TMHDI 24.53 | 1.4823 | 5.85 | 187 | 46 |
| | 6B | EGMA 11.55 | HEMA 14.70 | TMHDI 23.75 | 1.4807 | 5.82 | 184 | 41 |
| | 7B | EGMA 12.04 | HEA 13.67 | NBDI 24.29 | 1.5022 | 5.52 | 193 | 47 |
| | 8B | EGMA 11.65 | HEMA 14.84 | NBDI 23.51 | 1.5003 | 5.68 | 186 | 37 |
| | 9B | TMPDA 20.11 | HPA 12.23 | XDI 17.66 | 1.5205 | 5.47 | 189 | 42 |
| | 10B | PETA 22.30 | HPA 11.33 | XDI 16.37 | 1.5202 | 5.35 | 204 | 51 |
| | 11B | EGMA 11.37 | 4HBA 15.63 | NBDI 23.00 | 1.4999 | 5.81 | 180 | 34 |
| | 12B | EGMA 11.03 | HEA 12.55 | TMXDI 26.42 | 1.5138 | 5.23 | 201 | 45 |
| | 13B | EGMA 10.48 | 4HBA 14.11 | TMXDI 25.11 | 1.5106 | 5.47 | 198 | 38 |
| Comparative Examples | 1B | - | - | - | 1.5190 | 14.00 | 133 | 12 |
| | 2B | - | - | - | 1.4261 | 13.50 | 119 | 10 |
| | 3B | - | - | - | 1.4592 | 14.00 | 178 | 30 |

As shown in Table 2, Examples using a urethane allyl (meth)acrylate compound containing a urethane bond, an allyloxy group, and a (meth)acryloyloxy group were excellent in breaking strength and breaking energy as compared with Comparative Examples.

In addition, Examples were low and excellent in polymerization shrinkage rate as compared with Comparative Examples.

On the other hand, Comparative Example 1B and Comparative Example 2B having no (meth)acryloyloxy group were inferior in breaking strength and breaking energy. Comparative Example 1B and Comparative Example 2B were high and inferior in polymerization shrinkage rate.

The disclosures of Japanese Patent Application No. 2019-148911 filed on August 14, 2019 and Japanese Patent Application No. 2019-199162 filed on October 31, 2019 are incorporated herein by reference in their entirety.

All documents, patent applications, and technical standards described in this specification are incorporated herein by reference to the same extent as when each individual document, patent application, and technical standards were specifically and individually indicated to be incorporated herein by reference.

(Note) Aspects of the disclosure include the following aspects.

<1A> A urethane allyl compound having a urethane bond and an allyloxy group.
<6A> A monomer composition containing a urethane allyl compound of the disclosure and a (meth)acrylate compound (D).
<8A> A molded body which is a cured product of the monomer composition of the disclosure.
<9A> A composition for a dental material containing the monomer composition of the disclosure and a polymerization initiator.
<11A> A dental material which is a cured product of the composition for a dental material of the disclosure.

<1B> A urethane allyl (meth)acrylate compound containing a urethane bond, an allyloxy group, and a (meth)acryloyloxy group.
<7B> A monomer composition containing the urethane allyl (meth)acrylate compound of the disclosure.
<10B> A molded body which is a cured product of the monomer composition of the disclosure.
<11B> A composition for a dental material containing the monomer composition of the disclosure and a polymerization initiator.
<12B> A dental material which is a cured product of the composition for a dental material of the disclosure.

## Claims

1. A urethane allyl compound having a urethane bond and an allyloxy group.

2. The urethane allyl compound according to claim 1, which is a urethane allyl compound (X) containing no (meth)acryloyloxy group or a urethane allyl (meth)acrylate compound (Y) containing a (meth)acryloyl group.

3. The urethane allyl compound according to claim 2,

   wherein the urethane allyl compound (X) is a reaction product of an iso(thio)cyanate compound (A) having two or more iso(thio)cyanate groups and an alcohol compound (B) having an allyloxy group, and
   the urethane allyl (meth)acrylate compound (Y) is a reaction product of an iso(thio)cyanate compound (A) having two or more iso(thio)cyanate groups, an alcohol compound (B) having an allyloxy group, and an alcohol compound (C) having a (meth)acryloyloxy group.

4. The urethane allyl compound according to claim 3, wherein the iso(thio)cyanate compound (A) having two or more iso(thio)cyanate groups contains at least one selected from the group consisting of m-xylylene diisocyanate, 1,3-tetramethylxylylene diisocyanate, a mixture of 2,2,4-trimethylhexamethylene diisocyanate and 2,4,4-trimethylhexamethylene diisocyanate, a mixture of 2,5-bis(isocyanatomethyl) bicyclo[2.2.1]heptane and 2,6-bis(isocyanatomethyl) bicyclo[2.2.1]heptane, and isophorone diisocyanate.

5. The urethane allyl compound according to claim 3 or 4, wherein the alcohol compound (B) having an allyloxy group is at least one selected from the group consisting of the following compounds (B-1), (B-2), and (B-3):

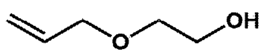

(B−1)

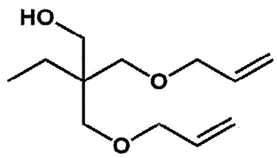

(B−2)

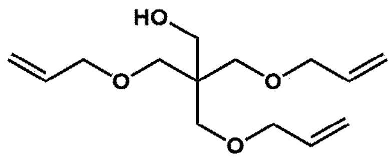

(B−3)

**6.** The urethane allyl compound according to any one of claims 3 to 5, wherein the alcohol compound (C) having a (meth)acryloyloxy group contains at least one selected from the group consisting of 2-hydroxyethyl (meth)acrylate, 2-hydroxypropyl (meth)acrylate, 2-hydroxybutyl (meth)acrylate, 4-hydroxybutyl (meth)acrylate, 2-hydroxy-3-phenoxypropyl (meth)acrylate, and 1,4-cyclohexanedimethanol mono(meth)acrylate.

**7.** The urethane allyl compound according to any one of claims 1 to 6, which is a compound represented by the following general formulas (XI) or (Y1):

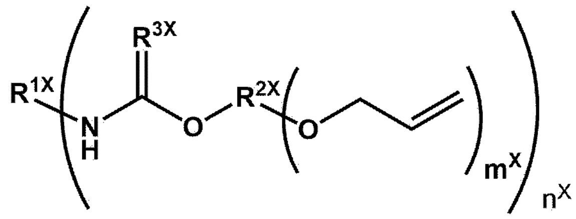

(X1)

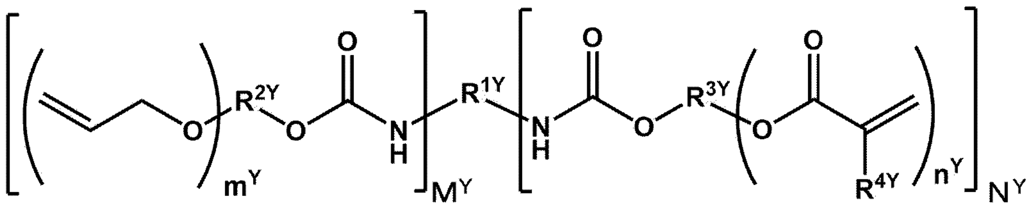

(Y1)

wherein, in formula (XI), $R^{1X}$ is a residue obtained by removing $n^X$ iso(thio)cyanate groups from the iso(thio)cyanate compound (A) having $n^X$ iso(thio)cyanate groups, $R^{2X}$ is a residue obtained by removing $m^X$ allyloxy groups and one hydroxy group from the alcohol compound (B) having $m^X$ allyloxy groups, $R^{3X}$ is an oxygen atom or a sulfur atom, $n^X$ is an integer of 2 or more, and $m^X$ is an integer of 1 or more,
and in formula (Y1), $R^{1Y}$ is a residue obtained by removing $M^Y + N^Y$ iso(thio)nate groups from the iso(thio)cyanate compound (A) having two or more iso(thio)cyanate groups,
$R^{2Y}$ is a residue obtained by removing $m^Y$ allyloxy groups and one hydroxy group from the alcohol compound (B) having an allyloxy group,
$R^{3Y}$ is a residue obtained by removing $n^Y$ (meth)acryloyloxy groups and one hydroxy group from the alcohol compound (C) having a (meth)acryloyloxy group, and
$R^{4Y}$ is a hydrogen atom or a methyl group, $n^Y$ is an integer of 1 or more, $m^Y$ is an integer of 1 or more, $M^Y$ is an integer from 1 to 3, $N^Y$ is an integer from 1 to 3, and $M^Y + N^Y$ is an integer from 2 to 4.

**8.** The urethane allyl compound according to any one of claims 1 to 7, wherein the urethane allyl compound has a molecular weight of from 200 to 1,500.

**9.** A monomer composition comprising the urethane allyl compound according to any one of claims 1 to 8 and a (meth)acrylate compound (D).

**10.** A monomer composition comprising the urethane allyl compound according to any one of claims 1 to 8, which is for

dental use.

**11.** A molded body which is a cured product of the monomer composition according to claim 9 or 10.

**12.** A composition for a dental material, comprising the monomer composition according to claim 9 or 10 and a polymerization initiator.

**13.** The composition for a dental material according to claim 12, further comprising a filler.

**14.** A dental material which is a cured product of the composition for a dental material according to claim 12 or 13.

FIG.1A

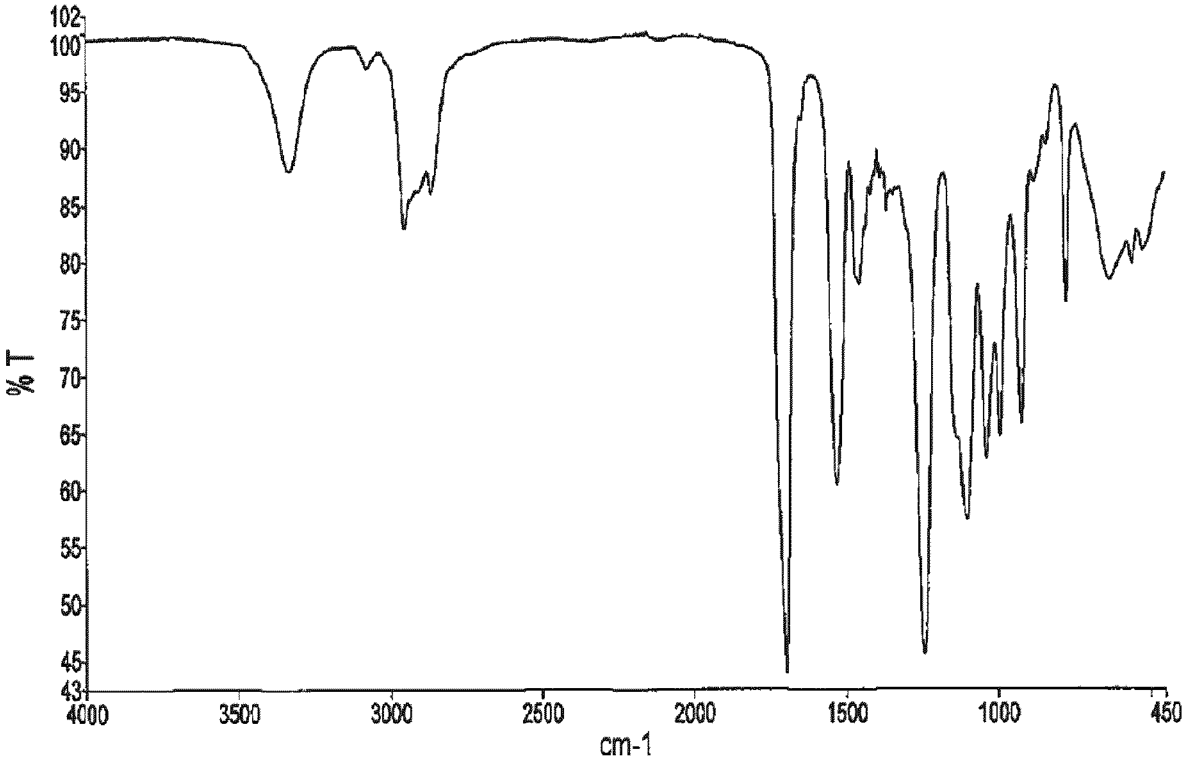
102
100
95
90
85
80
75
70
65
60
55
50
45
43
% T
4000
3500
3000
2500
2000
1500
1000
450
cm-1

FIG.1B

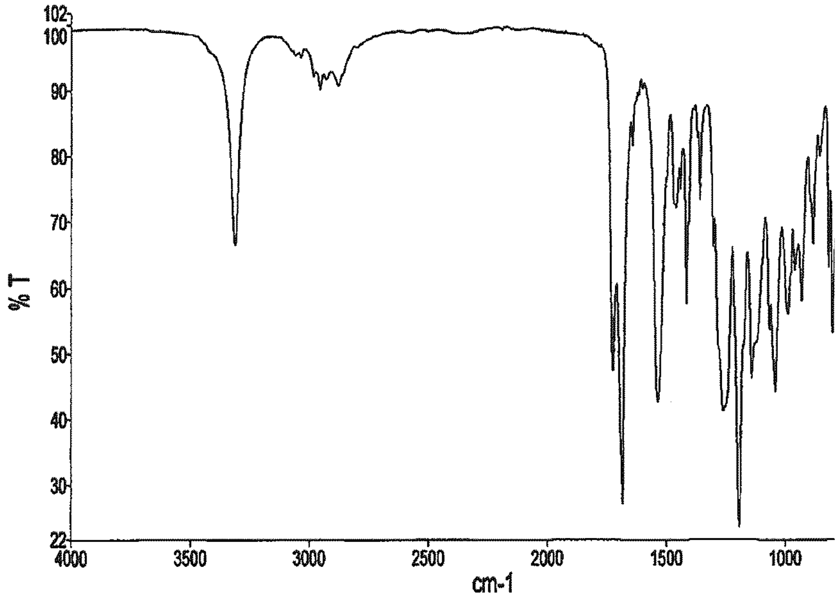
102
100
90
80
70
60
50
40
30
22
% T
4000
3500
3000
2500
2000
1500
1000
cm-1

FIG.2A

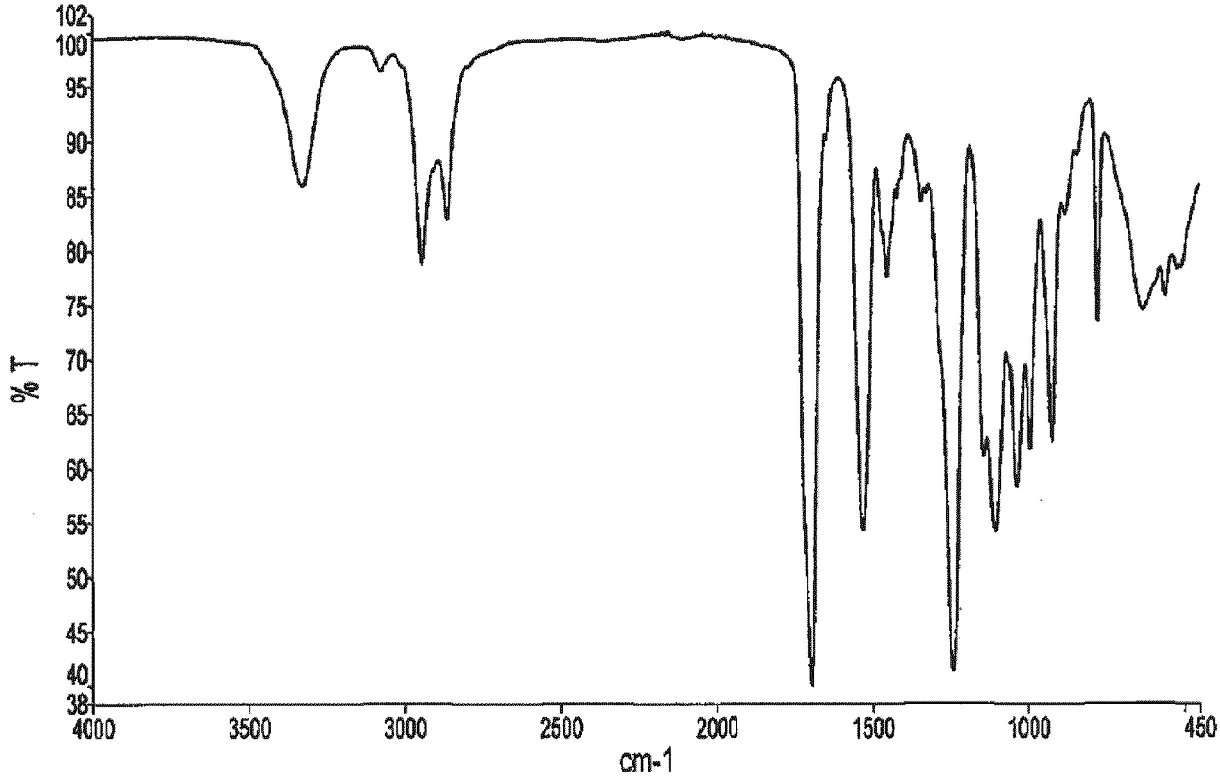
102
100
95
90
85
80
75
70
65
60
55
50
45
40
38
% T
4000
3500
3000
2500
2000
1500
1000
450
cm-1

FIG.2B

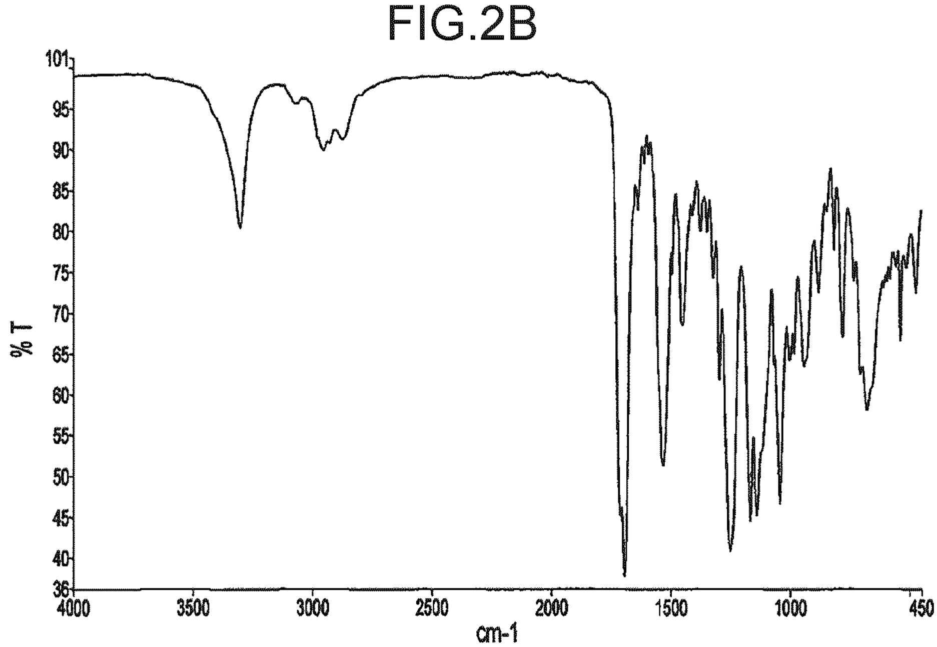
101
95
90
85
80
75
70
65
60
55
50
45
40
36
% T
4000
3500
3000
2500
2000
1500
1000
450
cm-1

FIG.3A

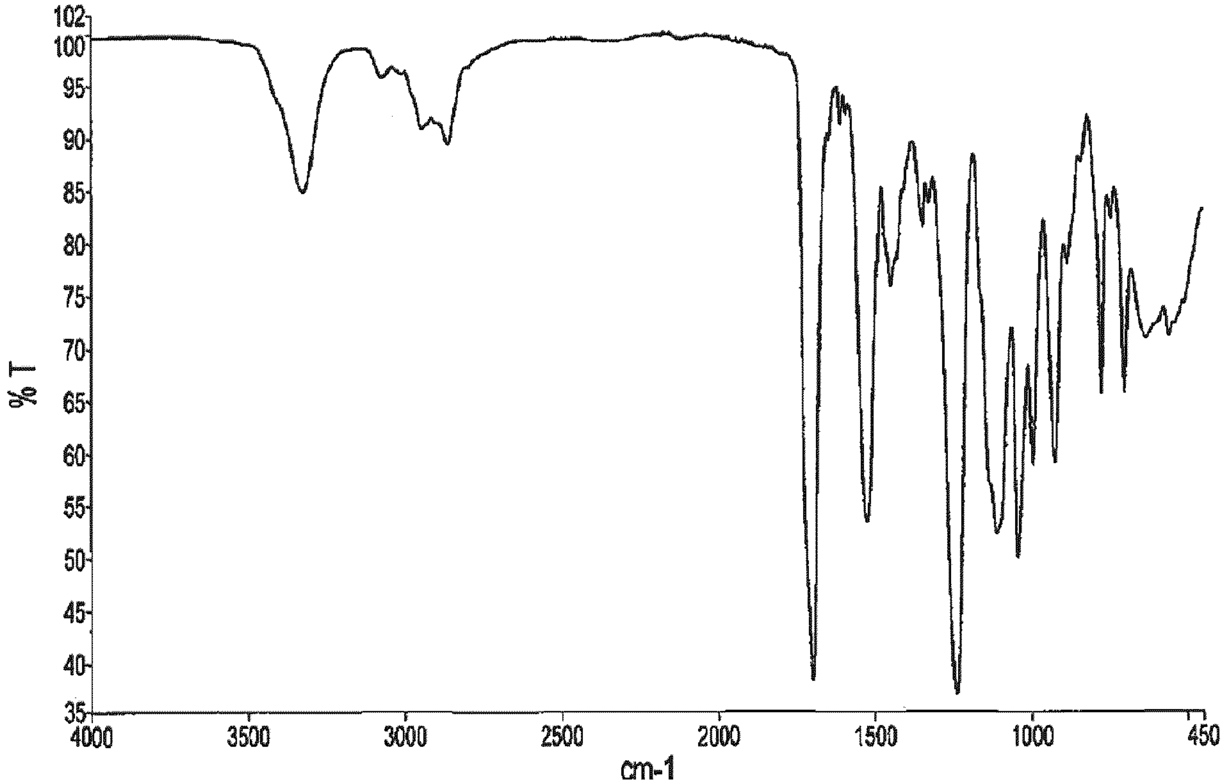
102
100
95
90
85
80
75
70
65
60
55
50
45
40
35
% T
4000
3500
3000
2500
2000
1500
1000
450
cm-1

FIG.3B

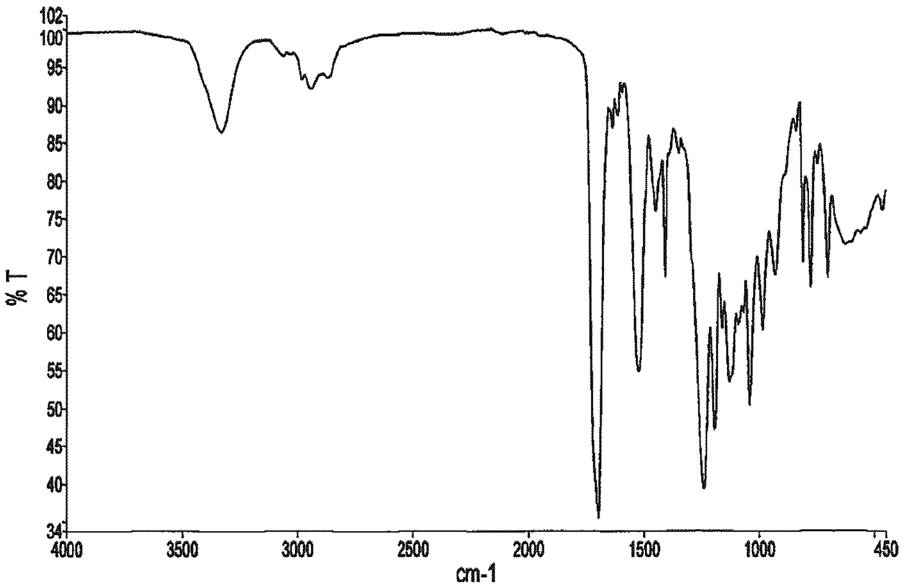
102
100
95
90
85
80
75
70
65
60
55
50
45
40
34
% T
4000
3500
3000
2500
2000
1500
1000
450
cm-1

FIG.4A

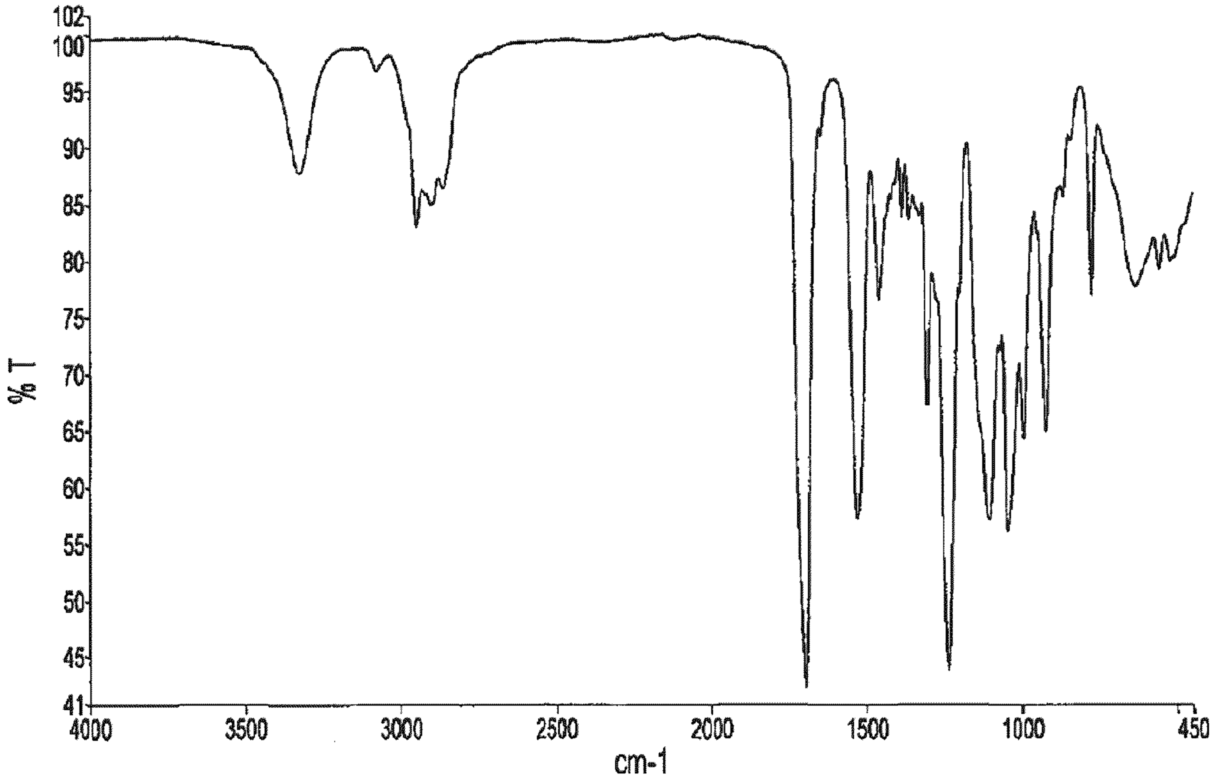
102
100
95
90
85
80
75
70
65
60
55
50
45
41
% T
4000
3500
3000
2500
2000
1500
1000
450
cm-1

FIG.4B

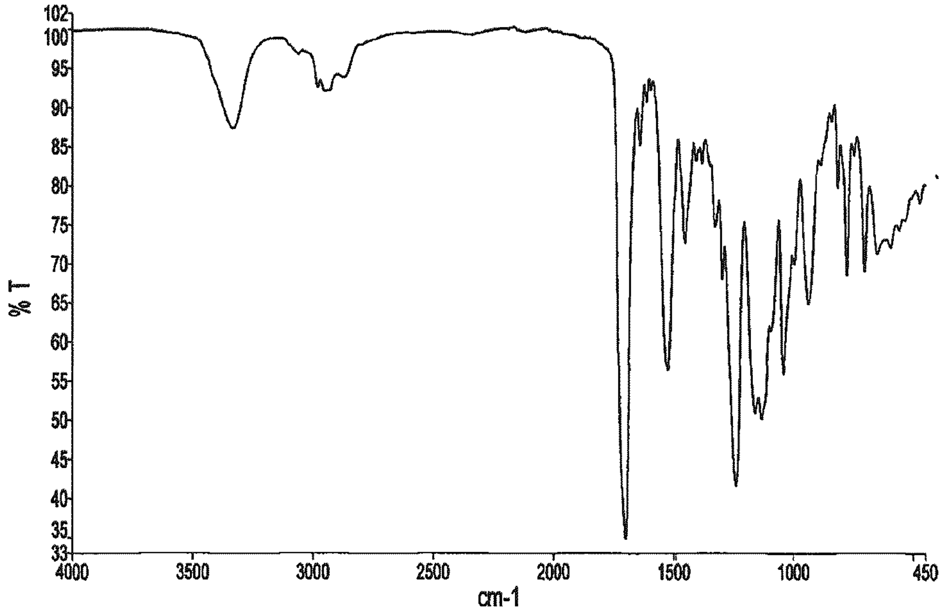
102
100
95
90
85
80
75
70
65
60
55
50
45
40
35
33
% T
4000
3500
3000
2500
2000
1500
1000
450
cm-1

FIG.5A

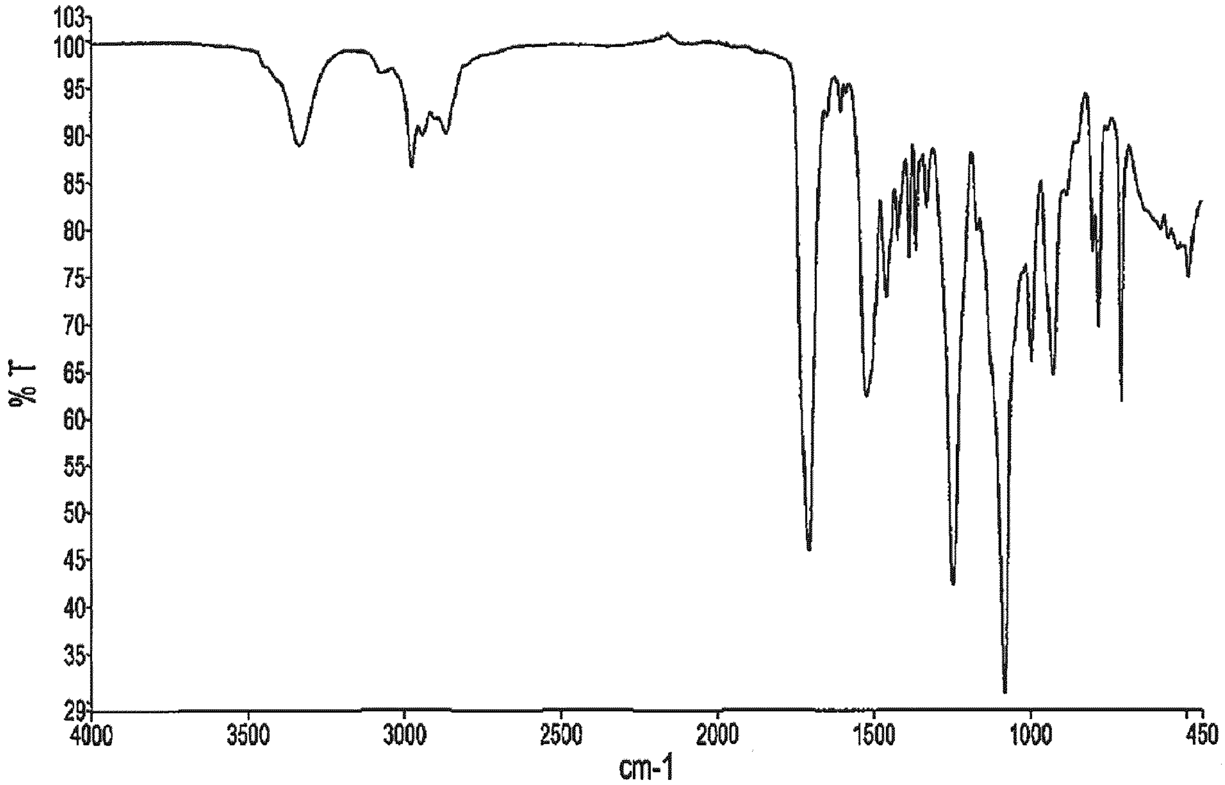
103
100
95
90
85
80
75
70
65
60
55
50
45
40
35
29
% T
4000
3500
3000
2500
2000
1500
1000
450
cm-1

FIG.5B

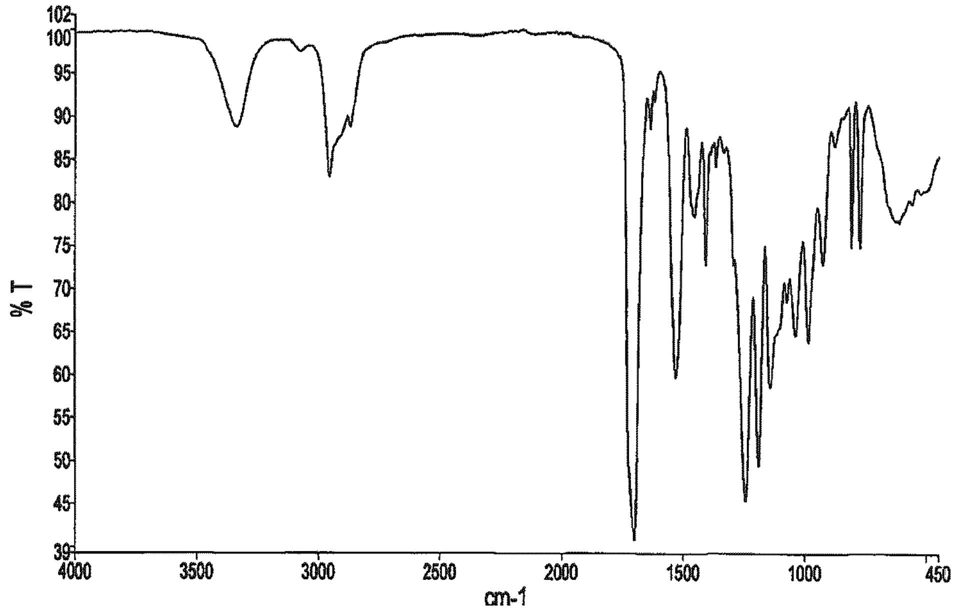
102
100
95
90
85
80
75
70
65
60
55
50
45
39
% T
4000
3500
3000
2500
2000
1500
1000
450
cm-1

FIG.6A

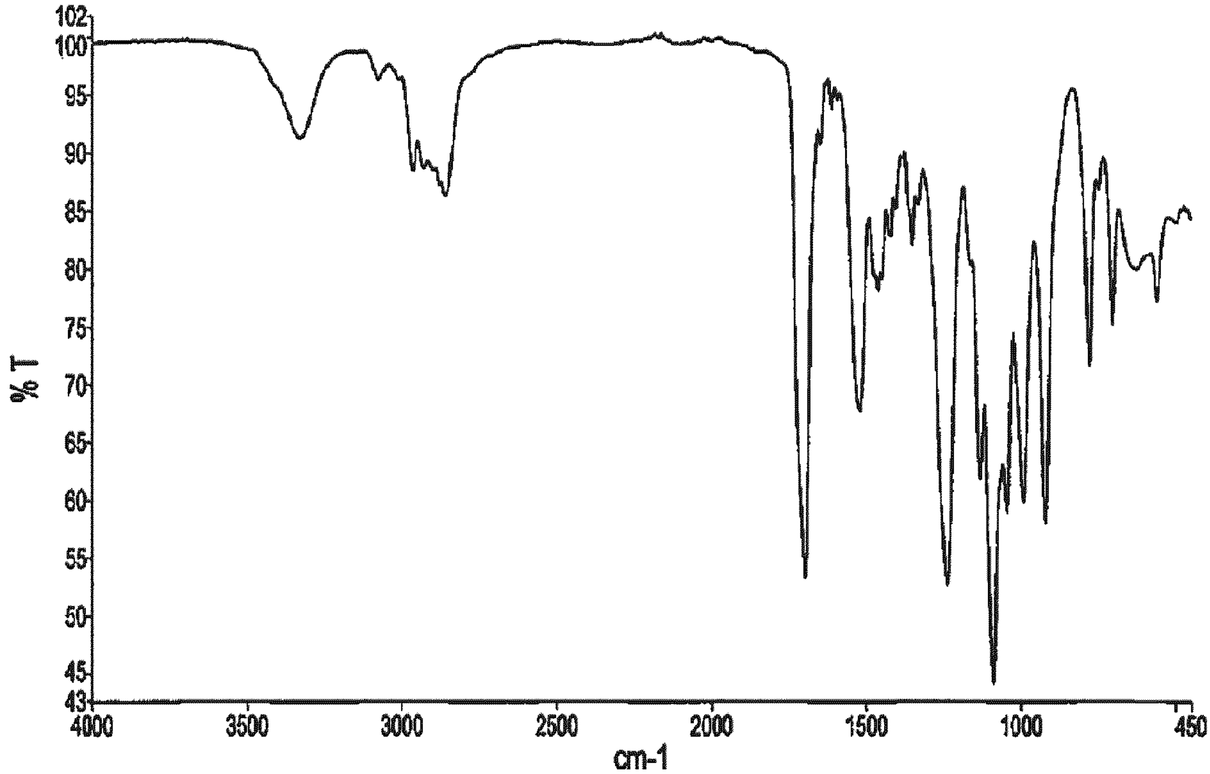
102
100
95
90
85
80
75
70
65
60
55
50
45
43
% T
4000
3500
3000
2500
2000
1500
1000
450
cm-1

FIG.6B

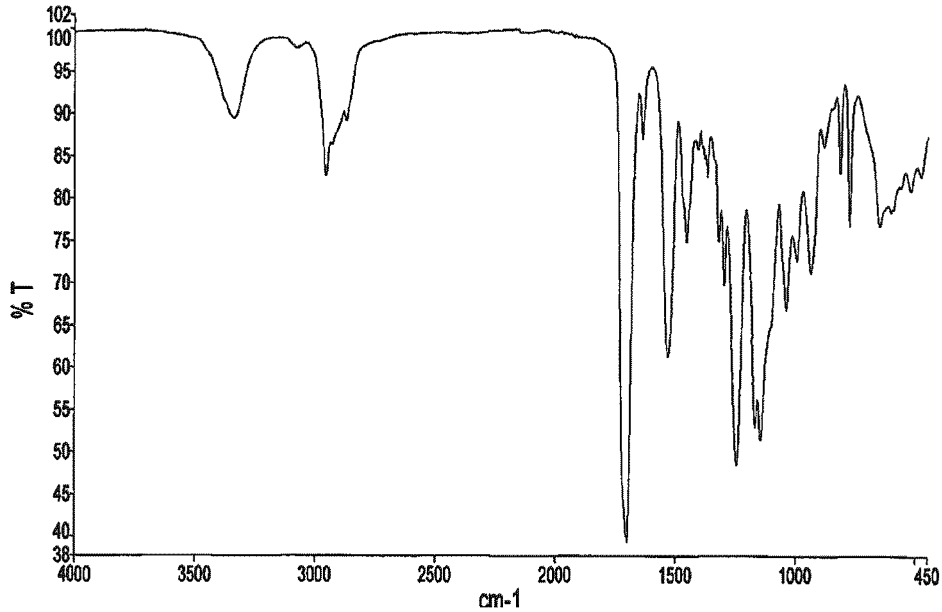
102
100
95
90
85
80
75
70
65
60
55
50
45
40
38
% T
4000
3500
3000
2500
2000
1500
1000
450
cm-1

FIG.7A

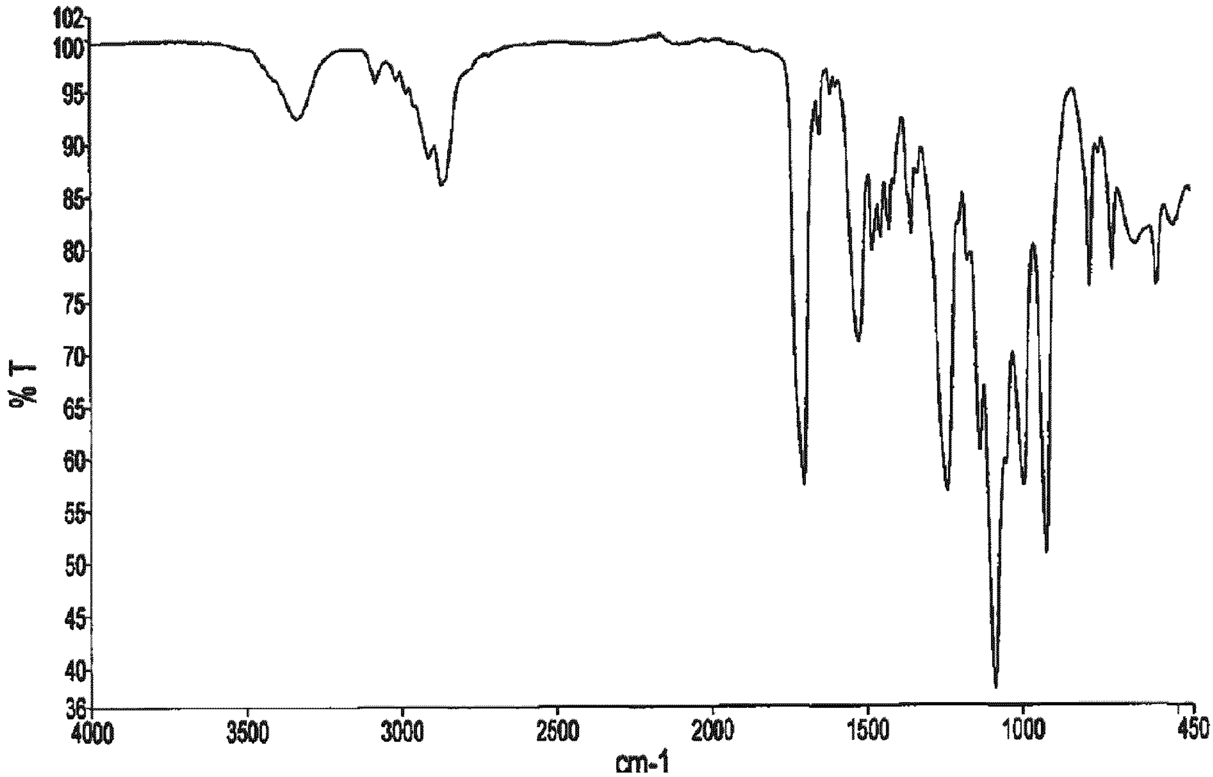
% T
cm-1
102
100
95
90
85
80
75
70
65
60
55
50
45
40
36
4000
3500
3000
2500
2000
1500
1000
450

FIG.7B

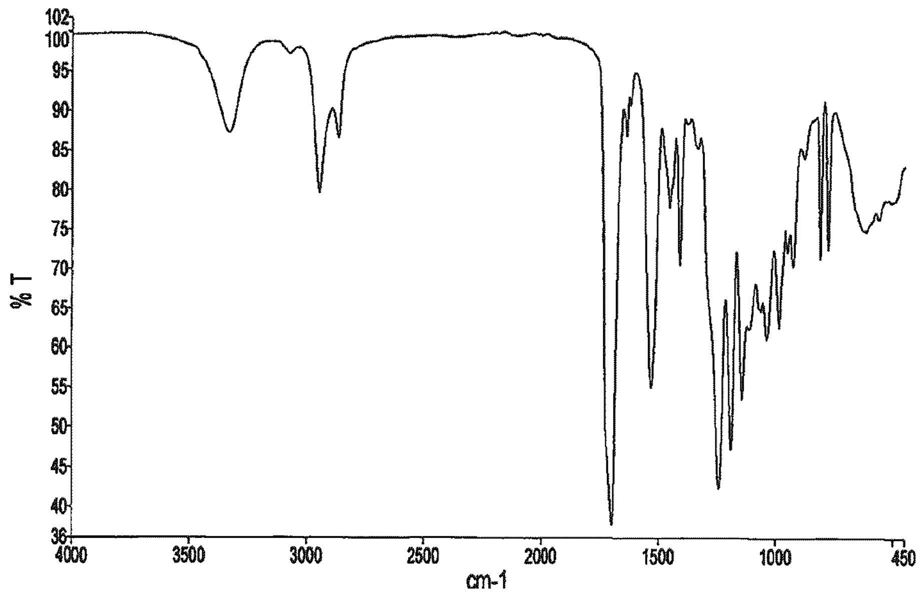
% T
cm-1
102
100
95
90
85
80
75
70
65
60
55
50
45
40
36
4000
3500
3000
2500
2000
1500
1000
450

FIG.8A

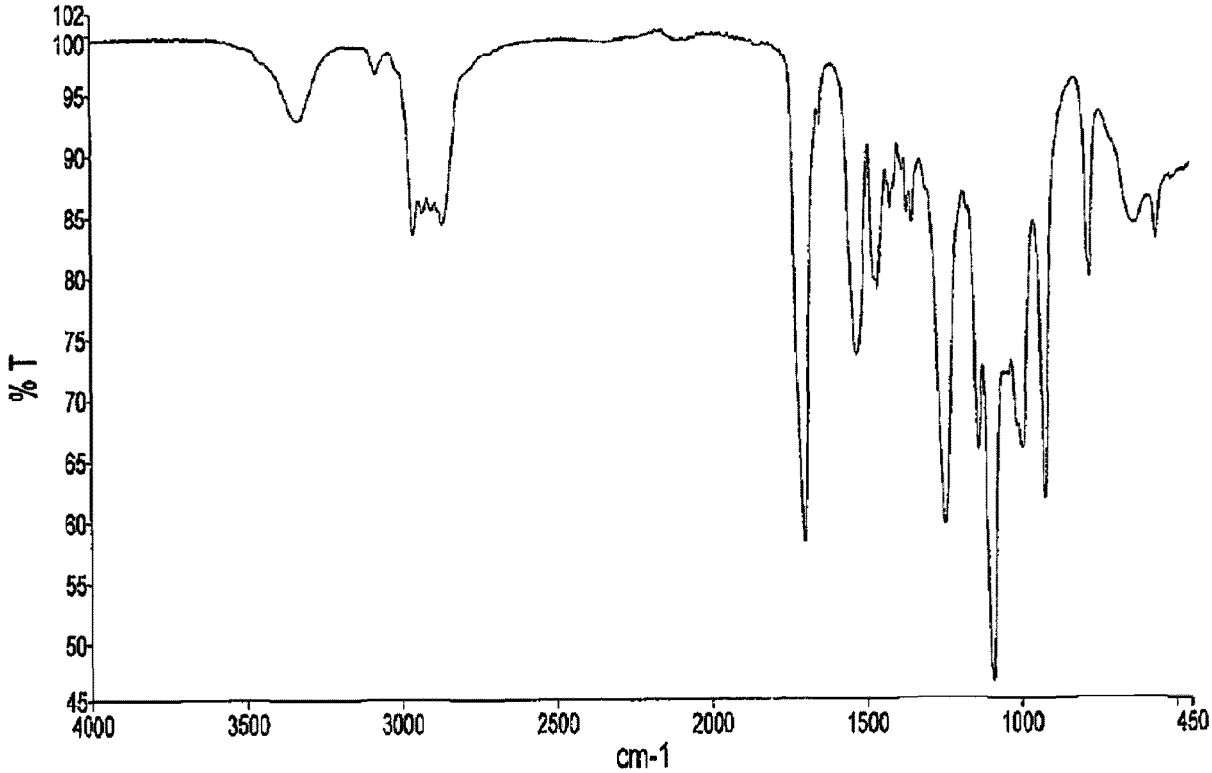
102
100
95
90
85
80
75
70
65
60
55
50
45
% T
4000
3500
3000
2500
2000
1500
1000
450
cm-1

FIG.8B

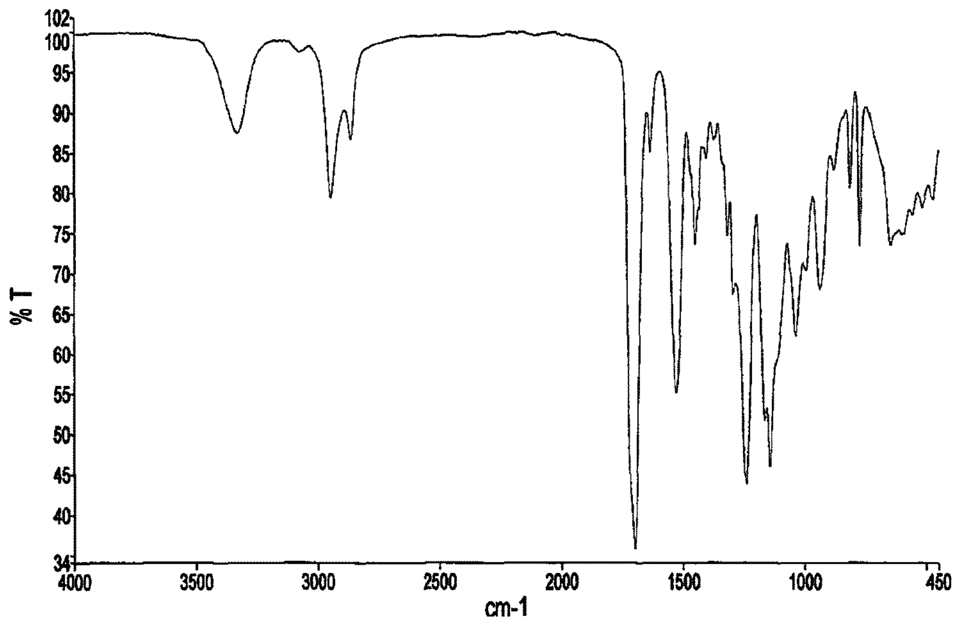
102
100
95
90
85
80
75
70
65
60
55
50
45
40
34
% T
4000
3500
3000
2500
2000
1500
1000
450
cm-1

FIG.9A

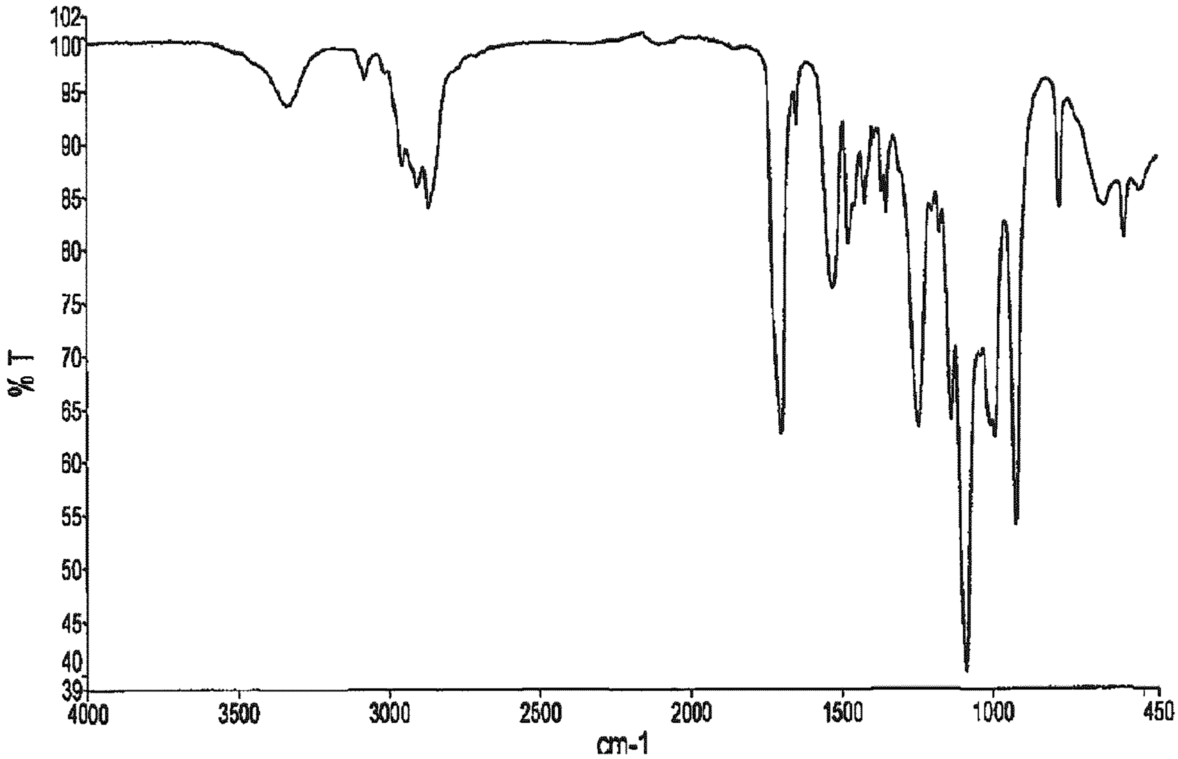
102
100
95
90
85
80
75
70
65
60
55
50
45
40
39
% T
4000
3500
3000
2500
2000
1500
1000
450
cm-1

FIG.9B

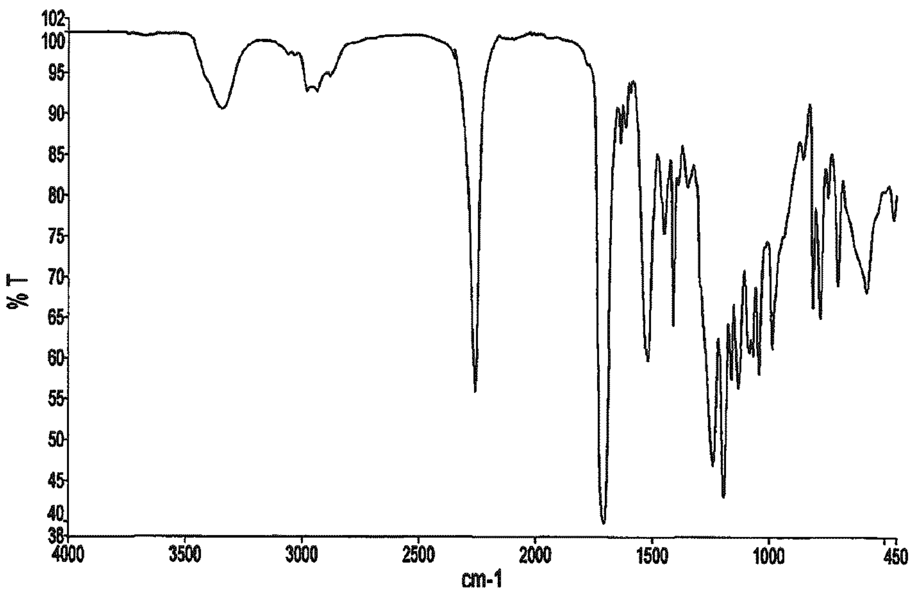
102
100
95
90
85
80
75
70
65
60
55
50
45
40
38
% T
4000
3500
3000
2500
2000
1500
1000
450
cm-1

FIG.10A

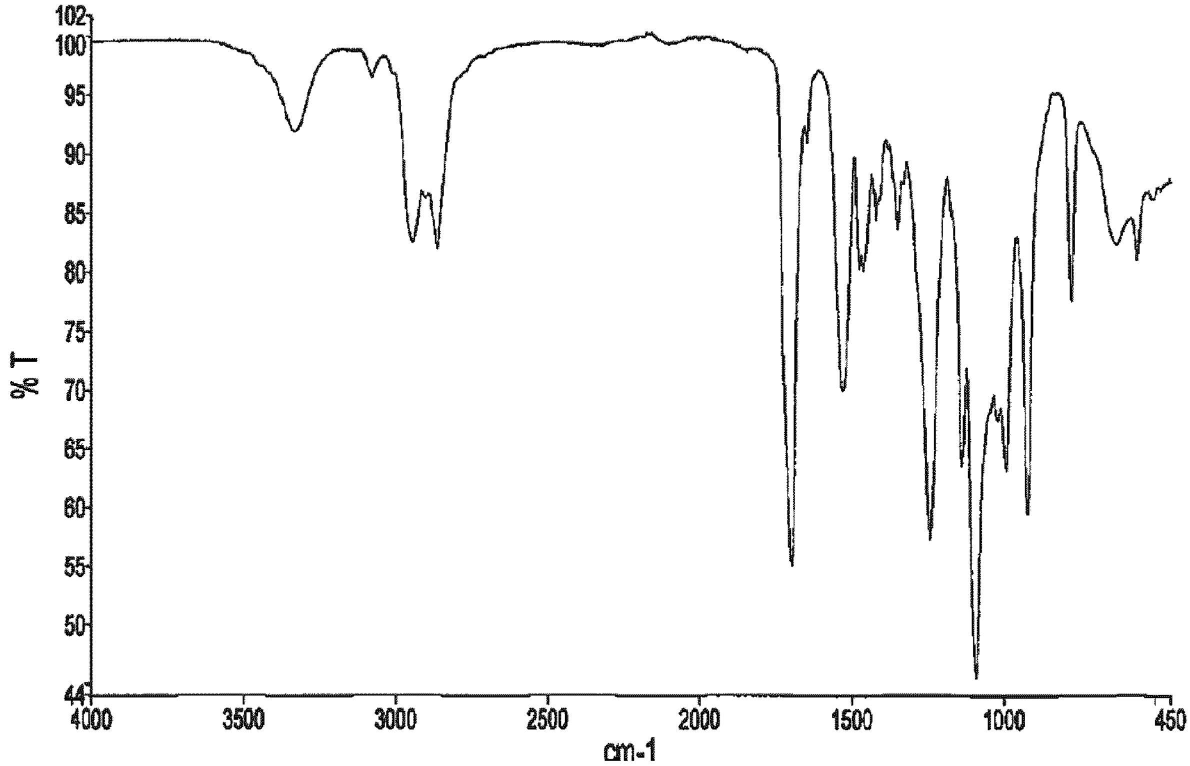


FIG.10B

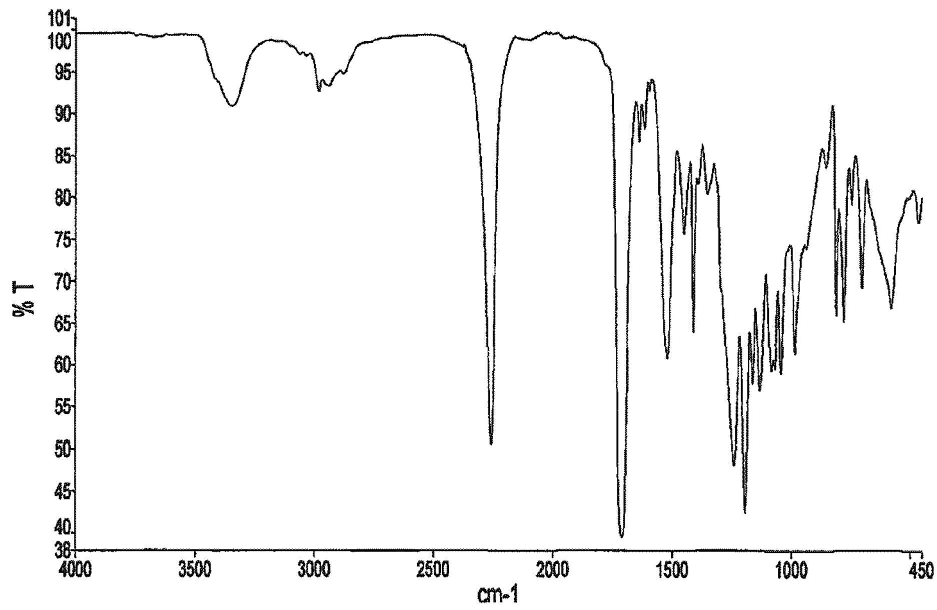

FIG.11A

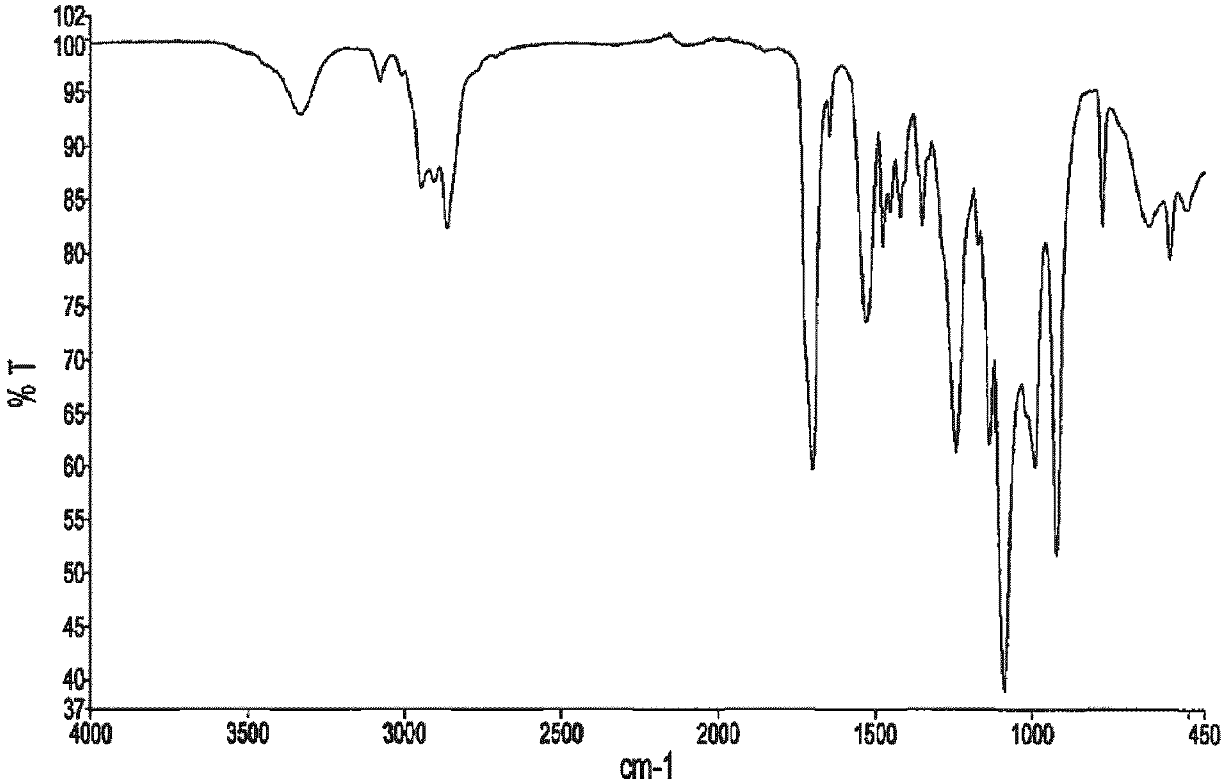


FIG.11B

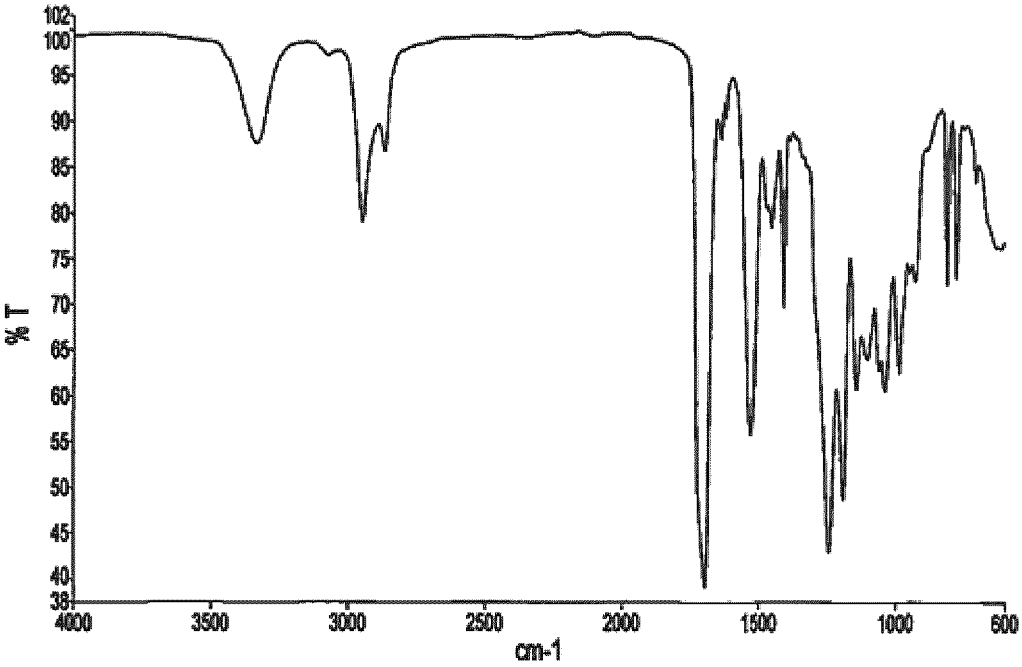

FIG.12B

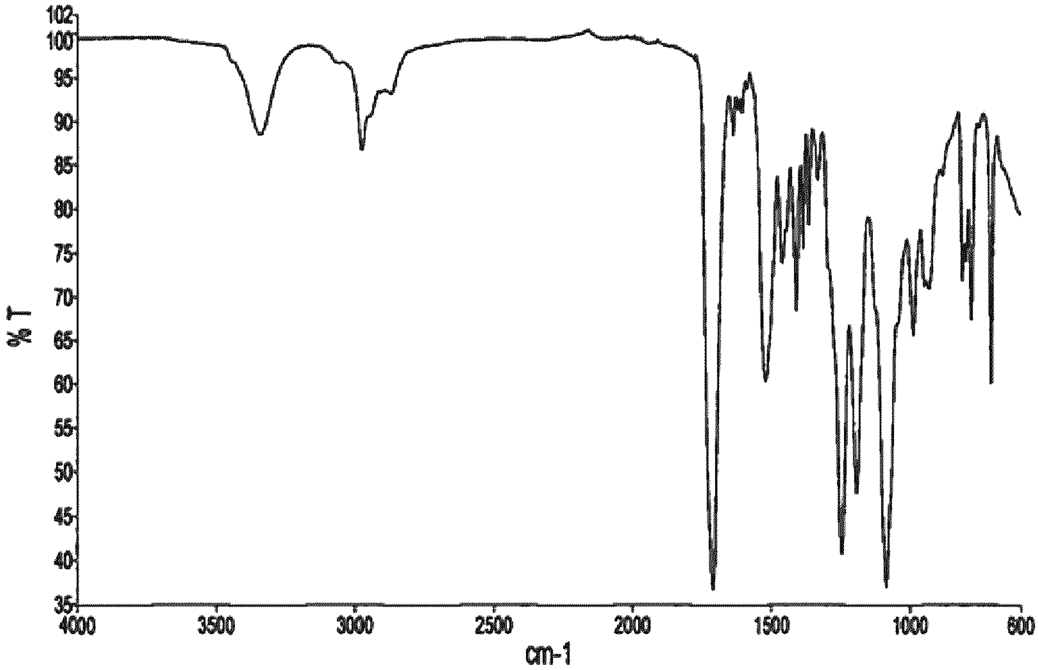


FIG.13B

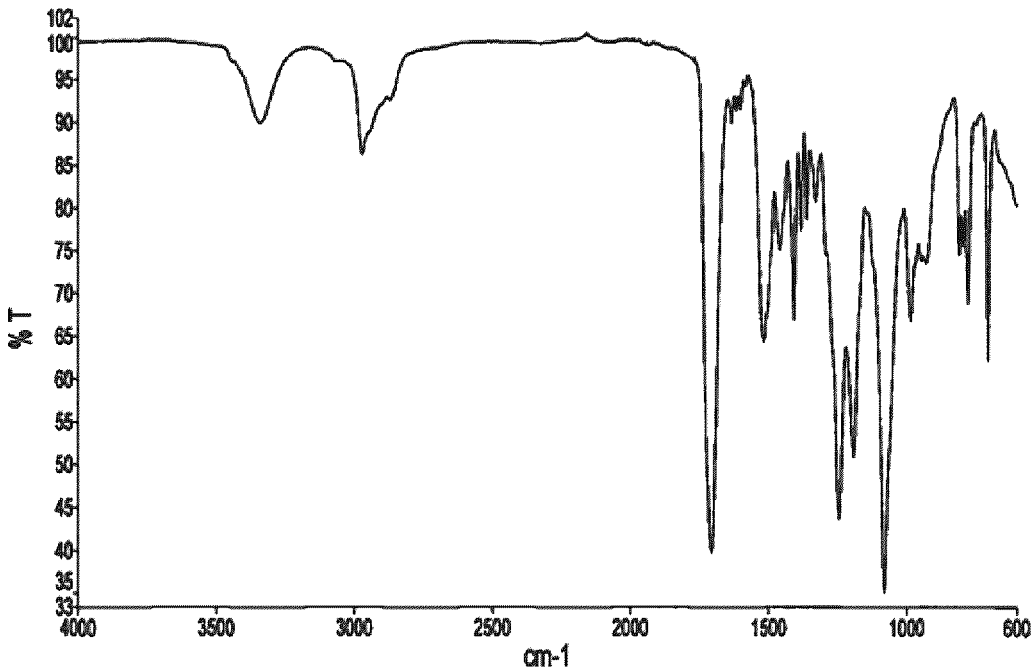

## INTERNATIONAL SEARCH REPORT

International application No.
PCT/JP2020/030607

A. CLASSIFICATION OF SUBJECT MATTER
C07C 271/10(2006.01)i; C07C 271/20(2006.01)i; C07C 271/24(2006.01)i; C08F 290/06(2006.01)i; C08G 18/67(2006.01)i; A61K 6/30(2020.01)i; A61K 6/882(2020.01)i; A61K 6/887(2020.01)i; A61K 6/889(2020.01)i; A61K 6/893(2020.01)i; A61K 6/90(2020.01)i
FI: C07C271/20 CSP; A61K6/887; A61K6/893; C07C271/24; C08F290/06; A61K6/30; A61K6/882; A61K6/90; C08G18/67 010; A61K6/889; C07C271/10 CSP

According to International Patent Classification (IPC) or to both national classification and IPC

B. FIELDS SEARCHED

Minimum documentation searched (classification system followed by classification symbols)
C07C271/00; C08F290/00; C08G18/00; A61K6/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X | PODGORSKI, Maciej et al., "Ester-free thiol-ene dental restoratives-Part A: Resin development", Dental Materials, 2015, 31(11), pp. 1255-1262, abstract, fig. 1, TENE, table 5 | 1-14 |
| X | BEIGI, Burujeny et al., "Assessments of antibacterial and physico-mechanical properties for dental materials with chemically anchored quaternary ammonium moieties: Thiol-ene-methacrylate vs. conventional methacrylate system", Dental Materials, 2015, 31(3), pp. 244-261, abstract, scheme 1, UTAE | 1-14 |

☒ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 October 2020 (08.10.2020) | 27 October 2020 (27.10.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/030607

C (Continuation). DOCUMENTS CONSIDERED TO BE RELEVANT

| Category* | Citation of document, with indication, where appropriate, of the relevant passages | Relevant to claim No. |
|---|---|---|
| X | BEIGI, Saeed et al., "valuation of fracture toughness and mechanical properties of ternary thiol-ene-methacrylate systems as resin matrix for dental restorative composites", Dental Materials, 2013, 29(7), pp. 777-787, abstract, page 780, scheme 1 | 1-14 |
| X | MCNAIR, OLivia D. et al., "Characterization of mouthguard materials: A comparison of a commercial material to a novel thiol-ene family", Journal of Applied Polymer Science, 2014, 131(13), pp. 40402/1 to 40402/11, abstract, page 40402/3, scheme 2 | 1 |
| X | JP 2007-277309 A. (AICA KOGYO CO., LTD.) 25 October 2007 (2007-10-25) page 17, paragraph [0077] | 1 |
| X | WO 2009/010423 A1 (CTYEC SURFACE SPECIALTIES, S.A.) 22 January 2009 (2009-01-22) page 12, 4 compound | 1 |
| X | US 2007/0123642 A1 (XEROX CORPORATION) 31 May 2007 (2007-05-31) pp. 20, 21, 24, compounds | 1 |

Form PCT/ISA/210 (continuation of second sheet) (January 2015)

**INTERNATIONAL SEARCH REPORT**
Information on patent family members

International application No.
PCT/JP2020/030607

| Patent Documents referred in the Report | Publication Date | Patent Family | Publication Date |
|---|---|---|---|
| JP 2007-277309 A | 25 Oct. 2007 | (Family: none) | |
| WO 2009/010423 A1 | 22 Jan. 2009 | (Family: none) | |
| US 2007/0123642 A1 | 31 May 2007 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S62149608 A
- WO 2019107323 A
- WO 2020040141 A
- JP 2019148911 A
- JP 2019199162 A